(19) Europäisches Patentamt
European Patent Office
Office européen des brevets

(11) EP 4 435 099 A1

(12) EUROPEAN PATENT APPLICATION
published in accordance with Art. 153(4) EPC

(43) Date of publication:
**25.09.2024 Bulletin 2024/39**

(21) Application number: **22894805.5**

(22) Date of filing: **16.11.2022**

(51) International Patent Classification (IPC):
***C12N 9/64*** (2006.01) ***C12N 9/96*** (2006.01)
***A61K 38/48*** (2006.01) ***A61P 9/10*** (2006.01)
***A61P 7/02*** (2006.01) ***A61P 25/02*** (2006.01)
***A61P 13/12*** (2006.01)

(52) Cooperative Patent Classification (CPC):
**A61K 38/48; A61K 47/60; A61P 3/10; A61P 7/02; A61P 9/10; A61P 9/12; A61P 13/12; A61P 25/02; A61P 27/02; C07K 1/107; C12N 9/64; C12N 9/96**

(86) International application number:
**PCT/CN2022/132102**

(87) International publication number:
**WO 2023/088272 (25.05.2023 Gazette 2023/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.11.2021 CN 202111353294**

(71) Applicant: **Zonhon Biopharma Institute, Inc.**
**Changzhou, Jiangsu 213125 (CN)**

(72) Inventors:
- **MA, Bruce Yong**
  **hangzhou, Jiangsu 213125 (CN)**
- **WANG, He**
  **hangzhou, Jiangsu 213125 (CN)**
- **WANG, Jun**
  **hangzhou, Jiangsu 213125 (CN)**
- **GE, Chennan**
  **hangzhou, Jiangsu 213125 (CN)**
- **JIANG, Chenyang**
  **hangzhou, Jiangsu 213125 (CN)**
- **YAO, Xiang**
  **hangzhou, Jiangsu 213125 (CN)**
- **SUN, Fang**
  **hangzhou, Jiangsu 213125 (CN)**
- **ZHANG, Tao**
  **hangzhou, Jiangsu 213125 (CN)**
- **ZHUANG, Yu**
  **hangzhou, Jiangsu 213125 (CN)**
- **ZHANG, Menghan**
  **hangzhou, Jiangsu 213125 (CN)**

(74) Representative: **Axe PI**
**Les Meillières**
**504 Avenue Colonel Meyère**
**06140 Vence (FR)**

Remarks:
The complete document including Reference Table(s) and the Sequence Listing(s) can be downloaded from the EPO website

# (54) UNDERGLYCOSYLATED KALLIKREIN I, AND POLYETHYLENE GLYCOL MODIFIER THEREOF AND PHARMACEUTICAL USE THEREOF

(57) Provided are low glycosylated kallikrein I with no or a small amount of glycosylation at the NFS sequence and polyethylene glycol modified product and pharmaceutical applications. KLK1 with lower glycosylation at NFS is more active than KLK1 with higher glycosylation at NFS. A recombinant KLK1 mutant without N-glycosylation at the NFS sequence is also provided, containing only two N-glycosylation sites at NMS and NHT. Glycosylation of the recombinant KLK1 mutant is relatively more consistent, the molecular weight of the product is relatively more homogeneous, the yield is higher, the purification process is simpler, and the biological activity is higher, and the quality is more controllable.



Processed by Luminess, 75001 PARIS (FR)

## Description

### TECHNICAL FIELD

The present invention relates to low glycosylated kallikrein I (KLK1) and its polyethylene glycol modified product and pharmaceutical applications. In particular, the present invention relates to KLK1 with no glycosylation or a small amount of glycosylation at the NFS sequence, especially mutants at the NFS sequence, and their polyethylene glycol modified product and drug applications.

### BACKGROUND OF THE INVENTION

Kallikrein, also known as kallidinogenase, is a serine protease with two major classes: plasma kallikrein (PK) and tissue kallikrein (TK), both play important physiological roles. At present, it is believed that human tissue kallikrein is composed of at least 15 members (KLK1-KLK15). There are many studies on tissue kallikrein I(KLK1), which plays a series of biological roles by converting kininogens into kinins and acting on corresponding receptors. A large number of studies have shown that KLK1 plays a role in the nervous system, circulatory system, respiratory system, diabetes, cancer, kidney diseases and so on.

At present, KLK1 products in the global market have two types: one is pancreatic kallikrein I extracted from pig pancreas, such as Yikai of Changzhou Qianhong Pharmaceutical, but the raw materials are limited by animal sources. And the other is extracted from human urine, such as urinary kallikrein for injection from Guangdong Tianpu, but aslo the collection of human urine is difficult, and there is a risk of virus contamination. In addition, there is a Glu/Lys polymorphism in the No. 162 amino acid of urinary KLK1, and there are great challenges in drug quality control. In addition to the natural extraction of KLK1 mentioned above, there have been many studies on recombinant human kallikrein I, and the most advanced one is DiaMedica's DM199, which is currently under phase II/III clinical study for the treatment of stroke.

KLK1 is an enzyme with rich glycosylations, and the degree of glycosylation has an important effect on the activity of the enzyme. N-glycosylation is the most concerned glycosylation in glycoprotein drugs, where the sugar chain is linked to the free -$NH_2$ group of a specific asparagine in the nascent peptide chain. The N-glycosylation motif must be NXS or NXT, where N represents asparagine, S represents serine, T represents threonine, X represents other amino acids except proline. And asparagine that does not meet the above conditions can not be N-glycosylated. Different N-glycosylation motifs are located at different positions in the three-dimensional structure of glycoprotein. The structure of glycoprotein determines which glycosyltransferases can bind to the glycosylation sites and produce different glycosylations. However, the effect of different glycosylation levels of KLK1 on drug efficacy and safety is still inconclusive. The high glycosylated, medium glycosylated and low glycosylated porcine pancreatic kallikrein I were purified and isolated by CN107058269A. The low glycosylated porcine pancreatic kallikrein I had poor stability of enzyme activity, which could be removed by purification process to improve the biological activity of the product and reduce the side effects. CN101134952A, patent of Techpool Bio-Phama, separated the intermediate of urinary kallikrein I to obtain two components with high molecular weight and low molecular weight. The high molecular weight component was selected as the drug component. It was found that compared with human urinary kallikrein I containing mixture with high and low molecular weight, the side effects of human urinary kallikrein I with single high molecular weight were significantly reduced. Techpool Bio-Phama is also trying to express recombinant human kallikrein I in CHO, and its patent CN101134953A discloed high molecular weight KLK1 containing three glycosylation sites. US20130323222A, patent of DiaMedica, disclosed CHO expression of recombinant human kallikrein I, high and low glycosylated KLK1 were purified respectively, and there was no significant difference in the activity of the two in vivo. However, the composition with high and low glycosylated KLK1 in a ration about 1: 1 had higher activity than either high glycosylated KLK1 or low glycosylated KLK1 alone.

In addition, even if the best glycosylated KLK1 for drug production is determined, how to control the glycosylation level of glycoprotein is still an important technical problem in modern pharmaceutical production. It is usually necessary to optimize the recombinant expression and purification methods to maximize the attainment of the target glycosylated product. For example, CN101134953A used CHO to express recombinant KLK1, and the [0010] paragraph pointed out that "the glycosylation degree of the expression product is improved by optimizing the culture methods". CN101092598A was used to express recombinant KLK1 in P. pastoris. The molecular weight of the high glycosylated product was 32871.16D, and the molecular weight of the low glycosylated product was 28975.79D with higher proportion. The two components had approximate MW, and were separated by three-step purification.

In addition to the problem of glycosylation, current KLK1 products also have problems such as poor biological stability, short half-life, need for repeated administration, and certain immunogenicity of recombinant proteins. For example, DM199 is treated with an intravenous drop infusion within 24 hours of stroke onset, followed by subcutaneous injection every 3 days for 22 days. Clinical trials have shown that KLK1 can reduce the incidence of stroke in high-risk populations and prevent stroke recurrence. As a drug to improve the compensation of cerebral collateral circulation,

long-term and multiple administration of KLK1 is required to achieve the therapeutic effect. It is foreseeable that long-term administration of KLK1 will become an important way, while frequent administration will reduce patient compliance.

Pegylation is a technology that uses polyethylene glycol modifiers to chemically modify proteins and peptides. At present, more than a dozen PEGylated protein drugs have been marketed. PEG modification can increase protein solubility, improve protein stability, prolong the half-life in vivo, and reduce immunogenicity. Pegylated recombinant kallikrein I is rarely reported at home and abroad.

## SUMMARY OF THE INVENTION

The first technical issue addressed in this application is the choice of KLK1 glycosylation.

As mentioned in the background, on the one hand, the effect of different glycosylation of KLK1 on drug efficacy and safety is not conclusive. On the other hand, even if the optimal glycosylation of KLK1 is identified, controlling the glycosylation level of the glycoprotein usually requires extensive optimization in recombinant expression and purification methods to maximize the yield of the target glycosylated product.

Through characterization analysis, the applicant finds that human kallikrein I(hKLK1) has three glycosylation sites, namely N78(N in the NMS sequence), N84(N in the NHT sequence) and N141(N in the NFS sequence). All the three sites of high glycosylated KLK1 are glycosylated. In the low glycosylated KLK1, N78 and N84 is glycosylated, N141 is not glycosylated, or only a small amount of glycosylation. Further comparison of the biological activities of KLK1 with high and low glycosylation revealed that **the activity of low glycosylated KLK1 is much higher than that of high glycosylated KLK1.** Furthermore, in order to obtain low glycosylated KLK1, the applicant did not focus on the optimization of recombinant expression and purification methods as the conventional method, but on the basis of the study of high and low glycosylated KLK1, the applicant mutated one or more amino acids of the NFS motif, so that it does not constitute an N-glycosylated motif. Thus, more homogeneous and higher yield of low glycosylated hKLK1 mutant is obtained. **In surprise, the applicants found that the low glycosylated KLK1 mutant had further advantages in enzymatic properties and activity compared with the unmutated low glycosylated KLK1.**

There are a variety of natural variants (homologs) of hKLK1, such as Genbank accession numbers: AAA59455.1, NP002248.1, AAA36136.1, AAP35917, AAU12569, etc. In terms of composition, the variants of hKLK1 have the same number of amino acids, have only a few different amino acids (as shown in the table below). They are highly homologous, and have basically the same in vitro and in vivo activities. The authors also found that they had the same glycosylation sites at N78, N84 and N141, and the motifs of the N-glycosylation sites are the same (NMS, NHT and NFS).

Table 1 Individual amino acid differences of KLK1 variants

| KLK1 variants | Position of amino acid | | | | |
|---|---|---|---|---|---|
| | 90 | 115 | 121 | 162 | 164 |
| AAA59455.1 | D | V | **Q** | K | A |
| NP002248.1 | D | V | E | K | A |
| AAP35917 | D | V | **Q** | K | **V** |
| AAA36136.1 | D | V | **Q** | **E** | A |
| AAU12569 | **N** | **F** | **Q** | **E** | A |

In this application, one or more amino acids of the NFS motif are mutated on the basis of AAA59455.1 to obtain KLK1 containing only two N glycosylation sites (low glycosylated KLK1). As mentioned above, the amino acid and glycosylation sites of hKLK1 variants are highly consistent. Mutations in the corresponding positions (i.e., one or more amino acids in the NFS motif) of other hKLK1 variants can also achieve the same technical effect, that is, to obtain more homogeneous and low glycosylated hKLK1 with higher yield and better activity .

In addition, the inventors found that other primate KLK1 contains the same three N-glycosylation sites and the same motif as hKLK1, which is NMS, NHT, NFS, respectively. Such as NCBI Reference Sequence:XP_004061305. 1 (gorilla), XP_003916022.1(East African baboon), XP_003813685.1(bonobo), XP_002829661.3 (Sumatran orangutan), XP_032024960.1(white broded gibbon), etc. Mutations in the corresponding position (i.e., one or more amino acids of the NFS motif) in other primate KLK1 can also achieve the same technical effect, that is, to obtain more homogeneous and low glycosylated KLK1 with higher yield and better activity.

Based on the above findings, the present application provides low glycosylated KLK1 or derivative thereof, which is primate KLK1 and contains three N-glycosylation sites of native KLK1 at NMS, NHT, and NFS sequences, of which the asparagine at NFS has no glycosylation or a small amount of glycosylation. "low glycosylated" or "small amount

of glycosylation" means that the proportion of glycosylated asparagine at NFS is$\leq$ 10%, < 9%, < 8%, < 7%, < 6%, < 5%, < 4%, < 3%, < 2%, < 1%, < 0.5%, or $\leq$ 0.1%. In a particular embodiment, 96.39% of the asparagine at NFS in the low glycosylated KLK1 is unglycosylated and less than 4% is glycosylated, which is more active compared to the highly glycosylated KLK1 at NFS.

Further, the present application provides recombinant KLK1 mutant or derivative thereof, which is primate KLK1, and has only two N-glycosylation sites, and dose not have three N-glycosylation sites. The primates can be human or non-human primates, such as gorillas, East African baboons, bonobos, Sumatrans orangutans, white-browed gibbons, etc.

Preferably, this recombinant KLK1 mutant or derivative thereof retains the N-glycosylation at NMS, NHT in native KLK1 and does not contain the N-glycosylation at NFS in native KLK1.

Preferably, the N(asparagine) at NFS of KLK1 is mutated to any amino acid other than asparagine, that is, asparagine at position 141 is mutated to any amino acid other than asparagine, and 0, 1, or 2 amino acids of F and S at NFS are mutated to any other amino acid, so that the variant does not constitute N-glycosylation motif at the corresponding position. Thus, there is no glycosylation at position 141. In addition to obtain more homogeneous and higher yield of low glycosylated KLK1, the mutants have better activity than unmutated low glycosylated KLK1.

Preferably, F(phenylalanine) at NFS of KLK1 is mutated to proline, that is, phenylalanine at position 142 is mutated to proline, and 0, 1, or 2 amino acids of N and S at NFS are mutated to any other amino acid, so that the variant does not constitute N-glycosylation motif at the corresponding position. Thus, there is no glycosylation at position 141. Similarly, in addition to obtain more homogeneous and higher yield of low glycosylated KLK1, the mutants have better activity than unmutated low glycosylated KLK1.

Preferably, S(serine) at NFS of KLK1 is mutated to any amino acid other than serine or threonine, that is, serine at position 143 is mutated to any amino acid other than serine or threonine, and 0, 1 or 2 amino acids of N and F at NFS are mutated to any other amino acid. So that the variant does not constitute N-glycosylation motif at the corresponding position. Thus, there is no glycosylation at position 141. Similarly, in addition to obtain more homogeneous and higher yield of low glycosylated KLK1, the mutants have better activity than unmutated low glycosylated KLK1.

Specific examples include mutation of asparagine to four different types of amino acids, neutral polar amino acid such as glutamine (Gln), acidic amino acid such as aspartic acid (Asp), basic amino acid such as arginine (Arg), and aliphatic amino acid such as alanine (Ala). So that the variant does not constitute N-glycosylation motif at NFS and thus there is no glycosylation. In addition to obtain more homogeneous and higher yield of low glycosylated KLK1, the mutants have better activity than unmutated low glycosylated KLK1.

Preferably, the KLK1 is hKLK1, and the native hKLK1 amino acid sequence is shown as Genbank accession numbers :AAA59455.1, NP002248.1, AAA36136.1, AAP35917, AAU12569, etc.

As preferred, amino acid sequence of the KLK1 mutant is shown as SEQ ID No: 3, SEQ ID No: 4, SEQ ID No: 5, or SEQ ID No: 6.

The application also provides compositions containing the above low glycosylated KLK1.

KLK1 plays a series of biological roles by converting kininogens into kinins and acting on the corresponding receptors. A large number of studies have shown that KLK1 plays a role in the nervous system, circulatory system, respiratory system, diabetes, cancer, kidney diseases and so on. At present, KLK1 has been approved for microcirculation disorders, such as diabetic nephropathy, peripheral neuropathy, retinopathy, fundus disease, and adjuvant treatment of hypertension. It can also be used for mild to moderate acute thrombotic cerebral infarction. Other indications for clinical trials are IgA nephritis and chronic kidney disease. The KLK1 mutants in the present application have only a few amino acid mutations compared with the native KLK1, and the specific examples show that they all exert in vitro activity when acting on the same substrate, and also produce in vivo activity to improve the symptoms of cerebral infarction in the MACO cerebral ischemia-reperfusion model. Those skilled in the art can reasonably expect that the recombinant KLK1 mutants and their compositions have the same function as native KLK1 and can be used in the treatment, prevention, prognosis recovery, and prevention of recurrence of acute ischemic stroke, peripheral neuropathy, retinopathy, fundus disease, hypertension, diabetic nephropathy, IgA nephritis, and chronic kidney disease.

The second technical problem addressed in this application is that current KLK1 products have poor stability, short half-life, need for repeated administration, and the recombinant protein has certain immunogenicity.

The present invention provides pegylated KLK1 that significantly improves protein stability, reduces immunogenicity, improves in vivo pharmacokinetic properties of the protein, and retains protein activity.

Preferably, the PEG-modified KLK1 is the low glycosylated KLK1 described above.

Preferably, the KLK1 is recombinant KLK1 containing only two N-glycosylation sites or a derivative thereof, not containing three N-glycosylation sites.

Preferably, the polyethylene glycol modifier forms covalent coupling with the free amino group of the N-terminus or lysine of the KLK1 protein, as shown in the general formula (1-1 or 1-2), wherein R represents KLK1,

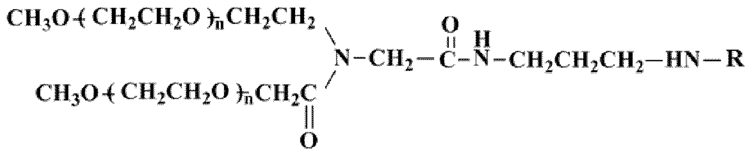

(1-1),

where n is an integer from 335 to 455;

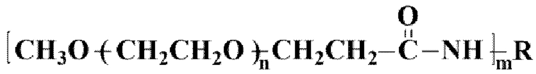

(1-2),

where n is an integer from 105 to 225 and m is an integer from 1 to 8.

Preferably, the PEG modifier is linear PEG succinimidyl propionate with molecular weight of 5kDa-10kDa, and the general formula is shown in (2),

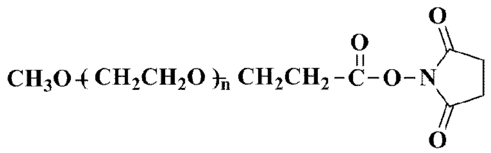

(2),

where n is an integer from 105 to 225,

Preferably, the polyethylene glycol modifier is branched polyethylene glycol propionaldehyde with molecular weight of 30 kda-40 kda, and the general formula is shown in (3),

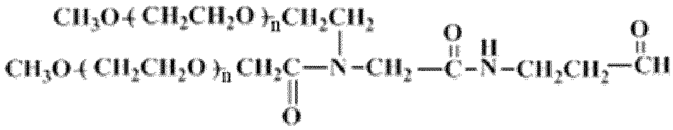

(3),

where n is an integer from 335 to 455.

The present invention provides preparation method of pegylated KLK1, comprising the following steps:

Step 1: Buffer displacement:
The KLK1 is replaced into the modification buffer by column desalting, dialysis, concentration and dilution, tangential flow ultrafiltration, etc.
Step 2: Modification reaction and purification of modified products

The reaction is performed after the addition of PEG modifier to the KLK1 solution collected in the first step, and the mixture is purified by ion exchange chromatography after the reaction.

The application also provides a composition containing the above pegylated KLK1.

The present invention also provides the application of the above-mentioned pegylated KLK1 in the treatment, prevention, prognosis recovery, and prevention of recurrence of acute ischemic stroke, peripheral neuropathy, retinopathy, fundus disease, hypertension, diabetic nephropathy, IgA nephritis, and chronic kidney disease.

The application has the following advantages over the prior art:
On the one hand, the advantages of low glycosylated KLK1 in this application are obvious.

First, low glycosylated KLK1 has no glycosylation or only a small amount of glycosylation at NFS, which has higher activity than high glycosylated KLK1.

Second, recombinant KLK1 mutants have further advantages:
The recombinant KLK1 mutant lacks one N-glycosylation site compared with the native KLK1. In terms of process, the purification process of recombinant hKLK1 mutant in this application only needs to collect the main peak, and the purification process is simpler.

In terms of quality, because there is no high and low glycosylated KLK1 that is difficult to separate, the target

product is relatively more homogeneous, more controllable in quality and higher in yield.

In terms of activity and efficacy, the application unexpectedly found that the low glycosylated KLK1 mutant has further advantages in enzymatic properties, activity, etc., compared with the unmutated low glycosylated KLK1.

On the other hand, the application develops pegylated recombinant hKLK1 with long-acting, controllable quality, low immunogenicity and high biological activity through pegylation technology. Through the study of drug efficacy and pharmacokinetics, the safety and long-acting of pegylated drugs are fully explored, the frequency of administration is reduced, and the compliance of patients is improved. Thus this application covers the prevention, treatment, prognosis recovery, and prevention of recurrence of acute ischemic stroke, peripheral neuropathy, retinopathy, ocular fundus disease, hypertension, diabetic nephropathy, IgA nephritis, chronic kidney disease and other diseases.

## BRIEF DESCRIPTION OF THE DRAWINGS

Figure 1: Molecular design of recombinant hKLK1 and mutants
Figure 2: Schematic diagram of the pZHK2.0 expression vector
Figure 3: Hydrophobic chromatogram of the fermentation broth supernatant of CHO cells expressed wild-type recombinant hKLK1. The arrow indicates the isolated high glycosylated product (left) and low glycosylated product (right).
Figure 4: SDS-PAGE electrophoretograms of high and low glycosylated proteins of CHO cells expressed wild-type recombinant hKLK1 after purification. Lane M represents the protein marker, lane 1 represents the high glycosylated hKLK1, and lane 2 represents the low glycosylated hKLK1.
Figure 5: Hydrophobic chromatogram of the fermentation brothsupernatant of CHO cells expressed hKLK1X1. The arrow indicates isolated single low glycosylated product.
Figure 6: SDS-PAGE electrophoretogram of low glycosylated recombinant hKLK1X1 expressed in CHO cells after purification. Lane M represents the protein marker and lane 1 represents the low glycosylated hKLK1 mutant.
Figure 7: SDS-PAGE electrophoretogram of CHO cells expressed wild-type recombinant hKLK1 containing unseparated high and low glycosylated protein. Lane M represents the protein marker, and lane 1 represents the mixture of high and low glycosylated wild-type recombinant hKLK1 .
Figure 8: Sequence Coverage map of the high glycosylated hKLK1 sample after trypsin digestion
Figure 9: Sequence Coverage map of the low glycosylated hKLK1 sample after trypsin digestion
Figure 10: The TIC spectrum of three batches of high glycosylated hKLK1 after trypsin digestion
Figure 11: The TIC spectrum of three batches of low glycosylated hKLK1 after trypsin digestion
Figure 12: Sequence Coverage map of hKLK1X1 sample after trypsin digestion
Figure 13: Anti-drug antibody detection in animals given PEG-hKLK1(low glycosylated)
Figure 14:Anti-drug antibody detection in animals given PEG-hKLK1(highly glycosylated)
Figure 15: Effect of the test article on neurological defect symptoms in example 10
Figure 16: Effect of the test article on cerebral infarction area in example 10
Figure 17: Brain slice map of the model group and the sham operation group in example 10
Figure 18: Brain slice map of urinary kallikrein group and pancreatic kallikrein I group in example 10
Figure 19: Effect of the test article on neurological defect symptoms in example 11
Figure 20: Effect of the test article on cerebral infarction area in example 11
Figure 21: Brain slice map of the model group and the sham operation group in example 11
Figure 22: Brain slice map of the drug administration group in example 11
Figure 23: Effect of the test article on neurological defect symptoms in example 12
Figure 24: Effect of the test article on cerebral infarction area in example 12
Figure 25: Brain slice map of model group, sham operation group and positive drug group in example 12
Figure 26: Brain slice map of different PEG modified hKLK1 in example 12
Figure 27: Effect of the test article on neurological defect symptoms in example 13
Figure 28: Effect of the test article on cerebral infarction area in example 13
Figure 29: Brain slice map of the model group and the sham operation group in example 13
Figure 30: Brain slice map of different PEG modified hKLK1 mutants in Example 13
Figure 31: Effect of the test article on neurological defect symptoms in example 14
Figure 32: Effect of the test article on cerebral infarction area in example 14
Figure 33: Brain slice map of the model group and the shame operation group in example 14
Figure 34: Brain slice map of drug administration group in example 14

## DETAILED DESCRIPTION OF THE INVENTION

Unless otherwise specified, the technical terms or abbreviations of this application have the following meanings:

Kallikrein I: Specifically tissue kallikrein I, KLK1.
hKLK1: unmutated human tissue kallikrein I with sequence homology to native human tissue kallikrein I; It covers various homologs of human KLK1, including but not limited to KLK1 as shown in Genbank accession numbers AAA59455.1, NP002248.1, AAA36136.1, AAP35917, AAU12569, etc.

Three glycosylation sites N78, N84 and N141 of KLK1: asparagine at position 78, 84 and 141 in KLK1 amino acid sequence, respectively. The corresponding N-glycosylation motifs are NMS, NHT, and NFS, with N for asparagine, M for methionine, S for serine, H for histidine, T for threonine, and F for phenylalanine.

High glycosylated hKLK1: unmutated hKLK1 with high glycosylation, that is, there are three glycosylation sites N78, N84 and N141 in high glycosylated hKLK1, each N-glycosylation are glycosylated.

Low glycosylated hKLK1: unmutated hKLK1 with low glycosylation, that is, there are three glycosylation sites N78, N84 and N141 in low glycosylated hKLK1, but no glycosylation, or only a small amount of glycosylation at N141 .

PEG-hKLK1(high glycosylated): polyethylene glycol modified high glycosylated hKLK1, said high glycosylated hKLK1 refers to the above unmutated hKLK1 with high glycosylation.

PEG-hKLK1(low glycosylated): polyethylene glycol modified low glycosylated hKLK1, said low glycosylated hKLK1 refers to the above unmutated hKLK1 with low glycosylation.

hKLK1X: hKLK1 mutant. hKLK1X1, hKLK1X2, hKLK1X3, and hKLK1X4 represent different mutants.

PEG-hKLK1X: Polyethylene glycol modified hKLK1 mutant.

KLK1 derivatives: It includes not only full-length protein of the KLK1 mutant described in this application, but also partial protein of the KLK1 mutant described in this application and the protein obtained by further mutation, fusion protein (including but not limited to albumin fusion, Fc fusion, etc.) and various forms of modification (except pegylated modification) on the basis of the KLK1 mutant described in this application.

Polyethylene glycol: PEG, usually formed by the polymerization of ethylene oxide, has branched, linear, and multi-arm forms. In general, those with molecular weight below 20,000 are referred to as PEG and those with larger molecular weight are referred to as PEO. Ordinary polyethylene glycol has one hydroxyl group at each end. If one end is blocked with methyl group, methoxy polyethylene glycol (mPEG) is obtained.

Polyethylene glycol modifiers: PEG modifiers refer to PEG derivatives with functional groups, which are activated polyethylene glycol and can be used for protein and peptide drug modification. The polyethylene glycol modifier used in this application was purchased from ZonHon Biopharma Institute,INC. or Beijing Jenkem Technology Co., LTD. The actual molecular weight of PEG modifier can be 90%-110% of the labeled value. For example, molecular weight of PEG5K may be 4.5-5.5 kDa.

PEG5K used specifically refers to M-SPA-5K, straight chain monomethoxy polyethylene glycol succinimidyl propionate with molecular weight of approximately 5kDa, with structure as shown in formula (1), where n is an integer from 105 to 110,

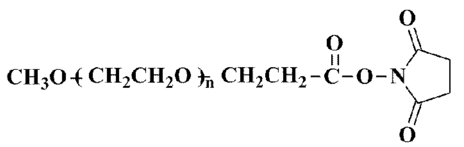

(1).

PEG10K used specifically refers to M-SPA-10K, straight chain monomethoxy polyethylene glycol succinimidyl propionate with molecular weight of about 10 kDa, with structure as shown in formula (1), where n is an integer from 220 to 225.

PEG30K used specifically refers to Y-PALD-30K, which is branched polyethylene glycol propionaldehyde with molecular weight of about 30kD, with structure as shown in formula (2), where m is an integer from 335 to 340,

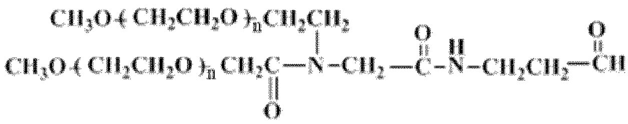

(2).

PEG40K used specifically refers to Y-PALD-40K, branched polyethylene glycol propionaldehyde with molecular weight of about 40kD, with structure as shown in formula (2), where m is an integer of 450 to 455.

**Example 1 Gene design, expression vector construction, expression, purification of recombinant human kallikrein I(hKLK1)**

(1). Gene design and expression vector construction

According to the available hKLK1 sequence in GenBank (GenBank:AAA59455.1), the mature amino acid sequence were determined (SEQ ID No: 1), the hKLK1 cDNA sequence(SEQ ID No: 2) encoding signal peptide and propeptide was obtained after codon optimization based on CHO system. AvrII sequence was added before the recombinant hKLK1 gene (SEQ ID No: 2), BstZ17I sequence was added after the sequence. The artificial sequence was synthesized and constructed into pUC57 plasmid to obtain long-term preservation plasmid, which was denoted as puc57-hKLK1 plasmid. Primers were used to amplify the hKLK1 gene from the pUC57-hKLK1 plasmid. The PCR products were purified after 1% agarose electrophoresis, and

The target gene PCR product(Figure 1) and pZHK2.0 vector (Figure 2) were both di gested with AvrII+BstZ17I and purified.. The target gene was ligated into the pZHK2.0 vector by T4 ligase. They were transformed into Top 10 competent cells and plated on LB plates containing kanamycin resistance for overnight culture at 37 ° C. The next day, positive clones were screened and sequenced, which were completely consistent with the expected sequence. The expression vector pZHK2.0-hKLK1 was formed.

(2) Stable expression

The constructed plasmid was linearized by overnight digestion with NruI(R0192S, NEB) and electrotransfected into CHO-S cells. Stable cell lines were obtained by pressure selection. The CHO cell line which can stablely and highly express recombinant hKLK1 was inoculated in Dynamis medium (A2617501, Thermo Fisher) at 37°C, 8% $CO_2$, 130rpm, fed-flow was added to the culture in batches and the culture medium was harvested after 2 weeks of culture.

(III) Purification

1. Pretreatment of culture medium

The above culture medium containing recombinant hKLK1 was collected, centrifuged at 6000rpm for 15min to remove cells, concentrated by ultrafiltration, and filtered through a 0.22μm membrane to remove cell debris. Then add 1.5M $(NH_4)_2SO_4$, and the mixture was activated by stirring at room temperature for 3 days. The activated culture medium was filterated through 0.45μm microfiltration membrane.

2. hydrophobic chromatography

The column was first equilibrated with buffer (20mM Tris-HCl, 1.5M ammonium sulfate, pH = 8.0) until baseline equilibrium. The pretreated supernatant was then run through POROS Benzyl medium (A32563, purchased from Thermobody) to capture recombinant hKLK1 from the medium. Then gradient elution was performed by the eluent(20mM Tris-HCl, pH = 8.0), and two obvious peaks through hydrophobic separation were observed. Each elution peak was collected. The recombinant hKLK1 proteins with inconsistent glycosylation were separated, and the chromatogram was shown in FIG. 3.

3. Anion exchange

The elution peak containing recombinant hKLK1 protein collected in step 2 was ultrafiltration with a 10kDa ultrafiltration membrane to a conductance of 10-15ms/cm with 20mM Tris-HCl, pH = 8.0 phosphate buffer. The column was equilibrated with 20mM Tris-HCl, 100mM NaCl, pH = 8.0 buffer until baseline equilibrium. The ultrafiltrated supernatant was then passed through Q FF medium (17515601, GE Company). Finally, the column was eluted equally with 20mM Tris-HCl, 1M NaCl, pH = 8.0 elution buffer, and the main elution peak was collected.

4. Cation exchange

Sample of step 3 was ultrafiltrated and changed to 10mM NaAc-HAc, 50mM NaCl, pH=3.5-3.7. Recombinant hKLK1 with different molecular weights collected in the previous step was purified with SP FF medium (17072904,

purchased from GE). Equilibration buffer was 10mM NaAc-HAc, 50mM NaCl, pH = 3.7, and elution buffer was 50mM Tris-HCl, 1M NaCl, pH 8.0.

The purity of the purified samples was analyzed by SDS-PAGE gel electrophoresis. The SDS-PAGE electrophoretogram showed that the molecular weight of the high glycosylated hKLK1 was slightly higher than that of the low glycosylated HKLK1, as shown in Figure 4.

**Example 2 Production and quality characterization of three continuous batches of recombinant human kallikrein I(hKLK1)**

Wild-type recombinant hKLK1 was cultured in three consecutive batches at high density in a 7L stirred reactor. The initial culture methods were as follows: 100rpm, 40% dissolved oxygen, 37°C, pH7.0, Dynamis AGT Medium(purchased from Thermo Fisher Scientific) was used as the basal medium, and the seed suspension was inoculated into the bioreactor at a density of $5\times10^5$cells/mL. On the third day, the temperature was adjusted to 33°C, and the feed medium (EfficientFeed C+AGT supplement, purchased from Thermo Fisher Scientific) and glucose were respectively supplemented to maintain the sugar content in the culture medium not less than 2g/L. When the cell viability was less than 90%, the culture medium was collected and purified using the purification process described in Example 1 to obtain three batches of recombinant hKLK1 with high and low glycosylation, respectively.

These samples were characterized, including yield, SEC-HPLC purity, amino acid sequence analysis of N/C terminus (Thermo Q Exactive), peptide mapping, glycosylation sites and glycosylation modification by LC/MS, and the results were summarized as follows.

Table 2

| Samples | Batch | Yield (mg) | SEC purity (%) | Amino acid sequence analysis of N/C terminus | Peptide mapping |
|---|---|---|---|---|---|
| high glycosylated hKLK1 | NO.1 | 139.62 | 98.95 | The N/C terminus were consistent with the theoretical sequence (Table 3) | Peptide map characterization of three batches were inconsistent (Fig. 10) |
| high glycosylated hKLK1 | NO.2 | 121.45 | 99.20 | Parts of N/C terminus was consistent with the theoretical sequence; Parts of C terminus was consistent with the theoretical sequence, while parts of the IVG sequence of N terminus was missing (table 4) | |
| high glycosylated hKLK1 | NO.3 | 129.00 | 100.00 | The N/C terminus were consistent with the theoretical sequence (Table 5) | |
| low glycosylated hKLK1 | NO.1 | 349.20 | 97.99 | The N/C terminus were consistent with the theoretical sequence (Table 6) | Peptide map characterization of three batches were consistent (Fig.11) |
| low glycosylated hKLK1 | NO.2 | 331.82 | 100.00 | The N/C terminus were consistent with the theoretical sequence (Table 7) | |
| low glycosylated hKLK1 | NO.3 | 290.10 | 100.00 | The N/C terminus peptides were consistent with the theoretical sequence (Table 8) | |

The analysis results in the above table showed that the purity of isolated high and low glycosylated recombinant hKLK1 is more than 97%. High and low glycosylated proteins had different molecular weights. Based on Trypsin digestion, the amino acid coverage of high and low glycosylated hKLK1 was detected, and the low glycosylated hKLK1 reached 100% coverage, and part of the high glycosylated hKLK1 reached 100% coverage, and N terminus of some high glycosylated hKLK1 was missing (Figure 8, 9). N/C terminus of the low glycosylated hKLK1 was consistent with the theory (Tables 6 to 8), and peptide map characterization of the three batches was consistent (Figure. 11). However, the N-terminus of some high glycosylated protein in batch 2 of three consecutive batches was inconsistent with the theoretical sequence and some N terminus sequence was missing(Table 3-5). The peptide map of batch 2 was inconsistent with

that of batch 1 and batch 3 (Figure 10), indicating that the high glycosylated recombinant hKLK1 may have molecular stability defects, which may bring huge risks in the process of drug production. At the same time, the separation and purification process of high and low glycosylated hKLK1 was also complex, and the instability of the process also made it difficult to control the glycosylation level of purified products among different batches.

Table 3 N/C terminus analysis results of the first batch 20200701 of high glycosylated hKLK1

| Peptide Sequence | Modification | Site | Delta (ppm) | RT (min) | M/Z | Charge State | Mono Mass Exp. | Avg Mass Exp. | Theoretical Mass | MS Area |
|---|---|---|---|---|---|---|---|---|---|---|
| N terminus : IVGGWECEQHSQ PWQAALYHFSTFQCGGILV HR | Amino methylation | **(C7,C26)** | **3.88** | **51.82** | **780.972** | **5** | **3897.8408** | **3900.09** | **3897.8257** | **2.48E+07** |
| C terminus : WIEDTIAENS | **None** | **** | **-2.26** | **26.06** | **1177.535** | **1** | **1176.5272** | **1177.11** | **1176.5299** | **3.74E+07** |

Table 4 N/C terminus analysis results of the second batch 20200703 of high glycosylated hKLK1 (IVG sequence of N-terminus missing)

| Peptide Sequence | Modification | Site | Delta (ppm) | RT (min) | M/Z | Charge State | Mono Mass Exp. | Avg Mass Exp. | Theoretical Mass | MS Area |
|---|---|---|---|---|---|---|---|---|---|---|
| N terminus : IVGGWECEQHSQ PWQAALYHFSTFQCGGILV HR | Amino methylation | **(C7,C26)** | **3.51** | **51.7** | **780.972** | **5** | **3897.8394** | **3900.09** | **3897.8257** | **1.07E+08** |
| N terminus: GWECEQHSQPW QALLYHFSTFQCGGILVHR | Amino methylation | **(C4,C23)** | **832** | **49.92** | **908.67** | **4** | **3628.682** | **3630.79** | **3628.652** | **9.17E+07** |
| C terminus : WIEDTIAENS | **None** | **** | **-2.88** | **26.02** | **1177.534** | **1** | **1176.5265** | **1177.11** | **1176.5299** | **7.33E+07** |

Table 5 N/C terminus analysis results of the third batch 20200705 of high glycosylated hKLK1

| Peptide Sequence | Modification | Site | Delta (ppm) | RT (min) | M/Z | Charge State | Mono Mass Exp. | Avg Mass Exp. | Theoretic al Mass | MS Area |
|---|---|---|---|---|---|---|---|---|---|---|
| N terminus : IVGGWECEQHSQ PWQAALYHFSTFQCGGILV HR | Amino methylation | **(C7,C26)** | **3.44** | **51.66** | **780.972** | **5** | **3897.8391** | **3900.09** | **3897.8257** | **1.53E+08** |
| C terminus : WIEDTIAENS | **None** | **** | **-1.54** | **26.03** | **1177.535** | **1** | **1176.5281** | **1177.11** | **1176.5299** | **5.45E+07** |

Table 6 N/C terminus analysis results of the first batch 20200702 of low glycosylated hKLK1

| Peptide Sequence | Modification | Site | Delta (ppm) | RT (min) | M/Z | Charge State | Mono Mass Exp. | Avg Mass Exp. | Theoretical Mass | MS Area |
|---|---|---|---|---|---|---|---|---|---|---|
| N terminus : IVGGWECEQHSQ PWQAALYHFSTFQCGGILV HR | Amino methylation | **(C7,C26)** | **5.01** | **51.57** | **780.972** | **5** | **3897.8452** | **3900.09** | **3897.8257** | **2.47E+08** |
| C terminus : WIEDTIAENS | **None** | **** | **-0.81** | **25.98** | **1177.536** | **1** | **1176.5289** | **1177.11** | **1176.5299** | **6.71E+07** |

Table 7 N/C terminus analysis results of the second batch 20200704 of low glycosylated hKLK1

| Peptide Sequence | Modification | Site | Delta (ppm) | RT (-in) | M/Z | Charge State | Mono Mass Exp. | Avg Mass Exp. | Theoretical Mass | MS Area |
|---|---|---|---|---|---|---|---|---|---|---|
| N terminus : IVGGWECEQHSQ PWQAALYHFSTFQCGGILVHR | Amino methylation | (C7,C26) | 4.45 | 51.6 | 780.973 | 5 | 3897.843 | 3900.1 | 3897.8257 | 2.34E+08 |
| C terminus : WIEDTIAENS | None | \ | -1.02 | 26 | 1177.536 | 1 | 1176.5287 | 1177.11 | 1176.5299 | 7.34E+07 |

Table 8 N/C terminus analysis results of the third batch 20200706 of low glycosylated hKLK1

| Peptide Sequence | Modification | Site | Delta (ppm) | RT (min) | M/Z | Charge State | Mono Mass Exp. | Avg Mass Exp. | Theoretic al Mass | MS Area |
|---|---|---|---|---|---|---|---|---|---|---|
| N terminus : IVGGWECEQHSQ PWQAALYHFSTFQCGGILV HR | Amino methylation | **(C7,C26)** | **4.26** | **51.7** | **780.972** | **5** | **3897.8423** | **3900.09** | **3897.8257** | **1.40E+08** |
| C terminus : WIEDTIAENS | **None** | **** | **-0.71** | **26.04** | **1177.536** | **1** | **1176.5291** | **1177.11** | **1176.5299** | **5.65E+07** |

Table 9 Glycopeptide characterization analysis results of N78/N84/ N141 sites in low glycosylated hKLK1(20200702)

| **Modification** | **Glycan** | **Category** | Sequence | **Confidence** | **Abundance** |
|---|---|---|---|---|---|
| -N78-A3Ga2G0F | A3Ga2G0F | N-Glycan | HNLFDDENTAQFVHVSESFPHPGFNMSLLENHTRQADEDYSHDLMLLR | 100 | 43.57% |
| ~N78-A4G3F | A4G3F | N-Glycan | HNLFDDENTAQFVHVSESFPHPGFNMSLLENHTRQADEDYSHDLMLLR | 100 | 56.43% |
| ~N84-A4GalG2F | A4GalG2F | N-Glycan | HNLFDDENTAQFYTWSESFPHPGFNMSLLENHTRQADEDYSHDLMLLR | 100 | 100% |
| N141+A1G0F | A1G0F | N-Glycan | VVELPTQEPEVGSTCLASGWGSIEPENFSFPDDLQCVDLK | 100 | 1.51% |
| N141+A1G0M4F | AlG0M4F | N-Glycan | VVELPTQEPEVGSTCLASGWGSIEPENFSFPDDLQCVDLK | 100 | 0.29% |
| N141+A2G0F | A2G0F | N-Glycan | VVELPTQEPEVGSTCLASGWGSIEPENFSFPDDLQCVDLK | 100 | 0.12% |
| N141+A2S2 | A2S2 | N-Glycan | VVELPTQEPEVGSTCLASGWGSIEPENFSFPDDLQCVDLK | 100 | 0.39% |
| N141+A3S2G0 | A3S2G0 | N-Glycan | VVELPTQEPEVGSTCLASGWGSIEPENFSFPDDLQCVDLK | 100 | 0.46% |
| N141+GnF | GnF | N-Glycan | VVELPTQEPEVGSTCLASGWGSIEPENFSFPDDLQCVDLK | 100 | 0.59% |
| N141+M3 | M3 | N-Glycan | VVELPTQEPEVGSTCLASGWGSIEPENFSFPDDLQCVDLK | 100 | 0.29% |
| N141+Unglycosylated | Unglycosylated | N-Glycan | VVELPTQEPEVGSTCLASGWGSIEPENFSFPDDLQCVDLK | 100 | 96.39% |

In addition, the analysis of the glycosylation sites of recombinant hKLK1 showed that N78 and N84 in recombinant low glycosylated hKLK1 were glycosylated, N141 was almost not glycosylated (96.39%), and only a small amount of N141 in hKLKl(less than 4%) was glycosylated (Table 9). The high glycosylated hKLK1 contains three N-glycosylation sites (N78, N84 and N141), and contains multiple types of glycosylation, and the types of glycosylation are complex.

High and low glycosylated protein abtained from the hydrophobic chromatography elution of Example 1 was combined, and the same anion and cation purification was performed to obtain recombinant hKLK1 protein with high and low glycosylation. The purity of the purified sample was analyzed by SDS-PAGE gel electrophoresis, shown in Figure 7. Bands of high and low glycosylated protein was diffused. A larger molecular weight component was present above the high glycosylated protein. As shown in Figure 3, the separation of unmutated hKLK1 with high and low glycosylation by hydrophobic purification was poor, and it was difficult to completely separate high and low glycosylated hKLK1. In conclusion, the unmutated hKLK1 proprotein was composed of high and low glycosylated hKLK1 with different molecular weights, and N141 contains inhomogeneous and complex glycosylation, which brought a great challenge for the quality control of recombinant hKLK1.

Further comparison of the biological activity of hKLK1 with high and low glycosylation (Example 6) revealed unexpectedly that the activity of hKLK1 with low glycosylation was much higher than that of hKLK1 with high glycosylation.

**Example 3 Design, expression vector construction, expression and purification of recombinant human kallikrein I mutant (hKLK1X)**

Further, in order to obtain low glycosylated hKLK1, the applicant did not strive to optimize the expression and purification methods of recombinant hKLK1 as the conventional method, but on the basis of the research on high and low glycosylated hKLK1, the applicant mutated one or more amino acids of the NFS motif, so that the corresponding position could not be glycosylated. Thus, more homogeneous and higher yield of low glycosylated hKLK1 mutant was obtained. In surprise, the low glycosylated hKLK1 mutant had further advantages in enzymatic properties and activity compared with the unmutated low glycosylated hKLK1.

The mutant exemplified in this example mutated N in the NFS motif (i.e., N141), and F and S in the NFS motif were not mutated. However, the embodiments were provided for illustrative purposes only and not to limit the scope of this application. All the following schemes can change the original NFS sequence so that it did not constitute N-glycosylation motif, thus there was no glycosylation at N141. For example N at NFS (i.e., N141) was mutated, 0, 1 or 2 amino acids of F and S at NFS were mutated to any other amino acid. Or F(F142) at NFS was mutated to proline, and 0, 1, or 2 amino acids of N and S at NFS were mutated to any other amino acid. Or S(i.e. S143) at NFS was mutated to any amino acid except threonine, and 0, 1, or 2 amino acids of N and F at NFS were mutated to any other amino acid. The above scheme not only obtained more homogeneous and higher yield of low glycosylated hKLK1, but also found that the obtained mutants had further advantages in enzymatic properties and activity compared with the unmutated product, which may be related to the lack of glycosylation at the original NFS sequence.

Further, this embodiment exemplized mutant based on the hKLK1 sequence shown in Genbank accession number AAA59455.1, but the embodiment was for illustrative purposes only and not to limit the scope of the application. The amino acids and glycosylation sites of other known natural variants of hKLK1 were highly consistent. Mutations in the corresponding position (i.e., one or more amino acids in the NFS motif) of other hKLK1variants can achieve the same technical effect, that was, to obtain more homogeneous and low glycosylated hKLK1 with higher yield and better activity. Other primate KLK1 contained the same three N-glycosylation sites as hKLK1, and the motif is the same, which is NMS, NHT, and NFS. The same technical effect can be achieved by mutating the corresponding position of other primate KLK1 (that was, one or more amino acids of the NFS motif). That was, more homogeneous and low glycosylated KLK1 with higher yield and better activity was obtained.

Specific mutant schemes exemplified in this embodiment were as follows:
Asparagine of the glycosylation site N141 was mutated to four different types of amino acids, namely neutral polar amino acid glutamine (Gln), acidic amino acid aspartic acid (Asp), basic amino acid arginine (Arg), and aliphatic amino acid alanine (Ala). The corresponding hKLK1 mutants were named hKLK1X1(SEQ ID No: 3), hKLK1X2(SEQ ID No: 4), hKLK1X3(SEQ ID No: 5), and hKLK1X4(SEQ ID No: 6), respectively. Each mutant was prepared as described in Example 1.

The recombinant hKLK1 mutant described above was purified using the three-step purification of Example 1. In the hydrophobic purification profiles of the mutants, only one main elution peak was observed in all cases. For example, the hydrophobic chromatogram of hKLK1X1 was shown in FIG. 5. This indicated that the product was more homogeneous after mutation. For unmutated recombinant hKLK1, the separation of high and low glycosylated hKLK1 was poor (Figure 3), and it was difficult to completely separate high and low glycosylated hKLK1. Hydrophobic chromatography of the mutant recombinant hKLK1 only need to collect one major peak and the purification process was simpler.

The purity of the purified samples was analyzed by SDS-PAGE gel electrophoresis, and it was found that the molecular weight of the purified mutant samples was very close to that of the unmutated low glycosylated hKLK1, and

the high glycosylated hKLK1 containing more N141 glycosylation completely disappeared (FIG. 6). The proportion of low glycosylated hKLK1 was higher, the yield was higher, and the product was more homogeneous.

The amino acid coverage detection of recombinant hKLK1X1 was completed by LC-MS, and the coverage rate was 100% with the theoretical amino acid sequence (FIG. 12), indicating that the primary sequence of recombinant hKLK1X1 was correct. The glycosylation sites were identified (Table 10). Recombinant hKLK1X1 contained only two glycosylation sites, N78 and N84, which were consistent with the goal of molecular design . Similar results were obtained for the other mutants.

Table 10 Identification of glycosylation sites in Chymotrypsin digested hKLK1X1-20210811 samples

| Peptide Sequence | Modification | Site | Delta (ppm) | RT (min) | M/Z | Charge State | Mono Mass Exp. | Avg Mass Exp. | Theoretical Mass | MS Area |
|---|---|---|---|---|---|---|---|---|---|---|
| VHVSESFP HPGFNMS | Nonspecific deamidation | N78 | 1.09 | 31.66 | 529.242 | 3 | 1584.7048 | 1585.64 | 1584.7031 | 1.15E+08 |
| ENHTRQAD EDYSHDLM | Nonspecific deamidation | N84 | 6.18 | 14.93 | 654.607 | 3 | 1960.7979 | 1961.82 | 1960.7857 | 4.76E+07 |

**Example 4 Deglycosylated mass determination of mutated and unmutated protein**

The deglycosylated mass of wild-type and mutated hKLK1 were detected by LC-MS after denaturation with guanidine hydrochloride and deglycosylation by PNGaseF. The molecular weight of high glycosylated hKLK1 after deglycosylation was 26380.338Da, which was basically consistent with the theoretical molecular weight of 26377.493Da (Table 11). The molecular weight of low glycosylated hKLK1 after deglycosylation was 26379.346Da, which was basically consistent with the theoretical molecular weight of 26377.493Da (Table 11). The molecular weight of hKLK1X1 after deglycosylation was 26418.82Da, which was basically consistent with the theoretical mass of 26418.48Da (Table 11). The molecular weights of hKLK1X2, hKLK1X3 and hKLK1X4 were also basically consistent with the theoretical mass. These results further confirmed the correctness of the primary structure of the recombinant protein.

Table 11 Summary of deglycosylated mass

| Sample name | Theoretical monoisotopic molecular weight (Da) | Measured monoisotopic molecular weight (Da) | ΔMass(Da) | Possible Modifications |
|---|---|---|---|---|
| high glycosylated hKLK1 | 26377.493 | 26380.338 | +2.845 | 3×deamidation |
| low glycosylated hKLK1 | 26377.493 | 26379.346 | +1.853 | 2×deamidation |
| hKLK1X1 | 26418.480 | 26418.82 | +0.34 | / |

**Example 5 Preparation, purification and purity analysis of PEG modified hKLK1/hKLK1X samples**

Different PEG modified hKLK1/hKLK1X can be prepared and purified by conventional methods, as exemplified in the following example.

1. Preparation of PEG-modified samples

(1) Pretreatment

The unmutated low glycosylated recombinant hKLK1 was treated with 3kDa ultrafiltration membrane package (or other equivalent buffer displacement method) with sodium dihydrogen phosphate/disodium hydrogen phosphate buffer(pH7.0) as the replacement buffer. And concentrate to 15mg/mL.

(2) Modification

PEG5K-hKLK1 was prepared by random modification: PEG was added to the pretreated hKLK1 protein solution according to 1:25 mass ratio of hKLK1 protein to M-SAP-5K PEG. The mixture was stirred slowly until it was evenly mixed, and the reaction was carried out for 24 hours at 4°C.

PEG10K-hKLK1 was prepared by random modification: PEG was added to the pretreated hKLK1 protein solution according to 1:20 mass ratio of hKLK1 protein to M-SAP-10K PEG, and the mixture was evenly mixed after slow stirring. The reaction was carried out for 24 hours at 4°C.

Preparation of site-modified PEG30K-hKLK1: PEG was added to the pretreated hKLK1 protein solution at 1:6 molar ratio of hKLK1 protein to Y-PALD-PEG 30K. Reducing agent was added to the mixture solution at 1:50 molar ratio of PEG to reducing agent (sodium cyanoborohydride). The mixture was slowly stirred until it was evenly mixed. The reaction was carried out for 24 hours at 4 °C.

Preparation of site-modified PEG40K-hKLK1: PEG was added to the pretreated hKLK1 protein solution at 1:6 molar ratio of hKLK1 protein to Y-PALD-PEG 40K. Reducing agent was added to the mixture solution at 1:50 molar ratio of PEG to reducing agent (sodium cyanoborohydride). The mixture was slowly stirred until it was evenly mixed. The reaction was carried out for 24 hours at 4°C.

2. Purification of the reaction mixture

The chromatographic conditions were as follows:
GE Q Sepharose™ High Performance medium was used as the purification filler, and the purification mobile phase was BufferA: 50mM Tris-HCl 9.0. BufferB: 50mM Tris-HCl+1M NaCl 9.0.

Loading: The above PEG-hKLK1 modification mixture after reaction was diluted about 10 times by double distilled water, and then diluted about 5 times by Buffer A solution before loading and purification. At the end of loading, the chromatographic column was washed with BufferA for more than 5 column volumes.

Elution: A gradient of 0-50% BufferB was set to elute 10 column volumes, elution samples were collected step by step according to UV280 trend.

PEG modified hKLK1 mutant (PEG-hKLK1X) was prepared and purified as described above.

3. Purity analysis of PEG modified samples

(1)HPLC purity analysis

The HPLC detection was carried out according to the General rule 0512 of Chinese Pharmacopoeia, 2020 edition. The chromatographic type was SEC(Size exclusion chromatography), the mobile phase was 20mM PB 7.0 containing 5% isopropanol, the chromatographic column was BEH450SEC 3.5μm, and the acquisition condition was 280nm. The acquisition time ranged from 20 to 25 minutes.

The results showed that the purity of the prepared PEG-hKLK1/hKLK1X series proteins was ≥ 95%.

(2) Purity analysis by SDS-PAGE

The purity of the samples was determined by SDS-polyacrylamide gel electrophoresis method, the fifth method of electrophoresis method in Chinese Pharmacopoeia (2020 edition), and 12.5% SDS-PAGE was used to detect the samples.

Electrophoresis results showed that the bands of PEG-hKLK1/hKLK1X series proteins were homogeneous,

no impurity bands were observed, and the purity was good.

(3)PEG binding number detection

a) Solution preparation: 1, 2, 4, 6, 8mg/ml PEG standard solution, 1mg/ml recombinant hKLK1 and corresponding PEG modified sample solution was prepared.
b) Detection method:

Column: XBridge BEH SEC 3.5μm 450A; Column temperature: 25 °C .
Mobile phase: 20mM PB 7.0+ 10% IPA, at a flow rate of 0.4ml/min.
Detector: PDA detector, detection wavelength 280nm; RI detector, detection wavelength 280nm.

c) Data analysis:

Measured concentration of test sample(PEG modified) = peak area detected by PDA detector of test sample(PEG modified)/peak area detected by PDA detector of unmodified protein × 1.0

PEG peak area in sample = (peak area of test sample(PEG modified) detected by RI detector/measured concentration of test sample (PEG modified))-(peak area of unmodified proprotein detected by RI detector/concentration of unmodified proprotein)

PEG peak area in the sample was substituted into the PEG standard curve to calculate PEG concentration in the sample.
PEG binding number in sample = (PEG concentration in sample/molecular weight of PEG)/(protein concentration in sample/molecular weight of protein 26kD)

Table 12

| **Sample name** | **measured concentration of test sample (mg/ml)** | **PEG peak area in the sample ($\mu$AU)** | **PEG concentration in sample (mg/ml)** | **PEG binding number** |
|---|---|---|---|---|
| PEG10K-hKLK1 | 0.96 | 319216 | 2.24 | 6.52 |
| (low glycosylated) | | | | |
| PEG10K-hKLK1X1 | 0.81 | 276707 | 1.93 | 6.24 |

**Example 6 In vitro activity detection of recombinant human kallikrein I(hKLK1) and its mutant (hKLK1X)**

1. In vitro activity evaluation based on artificial substrates

KLK1 exerts its biological function in vivo through catalyzing the hydrolysis of LMWK to release lysyl bradykinin, which involves the cleavage of the carboxy-terminus peptide bond of arginine (Arg). P-nitroaniline (PNA) was generated by hydrolyzating the amide bond between Arg and p-nitroaniline in the synthetic chromogenic substrate S-2266(H-D-Val-Leu-Arg-PNA). Therefore, PNA was detected at 405nm to evaluate the in vitro biological activity of recombinant hKLK1 and its mutants. One unit IU of activity was defined as the amount of enzyme that hydrolyzed 1$\mu$mol S-2266 to PNA per minute at 37°C and pH8.0. The reaction system consisted of 200gl 20mM trometamol buffer, 10$\mu$l test article, and 20$\mu$l 20mM S-2266 substrate solution, which were placed in water bath at 37°C for accurate reaction for 10min. The reaction was terminated by adding 20$\mu$l 50% acetic acid solution. The amount of PNA produced in the reaction system was quantified based on a standard curve fitted with different concentrations of PNA standards. The in vitro biological activities of hKLK1, hKLK1 mutants and hKLK1-PEG modified samples were detected by the above method.

As shown in the table below, the activity of low glycosylated hKLK1(unmutated) samples was significantly higher than that of high glycosylated hKLK1(unmutated) samples. Among the pegylated unmutated proteins, random modification had higher in vitro activity than site-directed modification, and PEG10K-hKLK1(low glycosylated) was better. After mutation, the activity of mutant samples (hKLK1X1, hKLK1X2, hKLK1X3, hKLK1X4) was higher than that of unmutated low glycosylated hKLK1 samples. At the same time, the activity of PEG modified samples was slightly lower than that of the unmodified hKLK1X1 protein. The activity of PEG10K-hKLK1X1 was higher than that of PEG5K-hKLK1X1, and the original protein activity was basically preserved after PEG10K modification.

Table 13

| Sample | Specific activity (IU/mg) | Relative activity |
|---|---|---|
| low glycosylated hKLK1 | 5.1 | 100% |
| high glycosylated hKLK1 | 3.0 | 58.8% |
| hKLK1X1 | 7.0 | 137.3% |
| hKLK1X2 | 7.1 | 139.2% |
| hKLK1X3 | 5.7 | 111.8% |
| hKLK1X4 | 5.7 | 111.8% |
| urinary kallikrein | 4.5 | 88.2% |

2. In vitro activity evaluation based on natural substrates

Enzymatic reaction and liquid phase detection

KLK1 exerts its biological function in vivo through catalyzing the hydrolysis of LMWK to release lysyl bradykinin. This example compared the enzymatic reaction to produce the effector molecule bradykinin at different ratios of substrate ( LMWK) to enzyme (KLK1 or its PEG modified KLK1). The produced effector molecules were separated by reverse phase chromatography, the peak area of the product is calculated, and the curve of bradykinin production at different ratios of substrate to enzyme was drawn. Compare the amount of effector molecules produced by different test samples under the same reaction methods. Through this in vitro method simulating the in vivo effect, the in vivo effects of different tested samples were indirectly compared.

hKLK1X1 was prepared according to Example 3, PEG10K-hKLK1X1 was prepared according to Example 5; Kb was KLK1 extracted from porcine pancreas. PEG-Kb was pancreatic kallikretin I for injection and was PEG10K-modified Kb, prepared as described in Example 5.

The samples were mixed according to the following table (in PEG modified samples, the molar mass of the modified active article was converted). The mixed samples were placed in a 37°C thermostat, incubated for 15min, accurately timed, and the reaction was terminated by adding 50% acetic acid solution according to the volume ratio of 10: 1.

Table 14

| System (substrate: enzyme mol/mol) | Substrate μl | Enzyme μl | 50% acetic acid μl | Total volume of system μl |
|---|---|---|---|---|
| 1: 1 | 10 | 10 | 2 | 22 |
| 5: 1 | 50 | 10 | 6 | 66 |
| 10: 1 | 50 | 5 | 5.5 | 60.5 |
| 15: 1 | 60 | 4 | 6.4 | 70.4 |
| 20: 1 | 60 | 3 | 6.3 | 69.3 |

After the termination of the reaction, the sample was placed in a desktop centrifuge, centrifuged at 12000rpm for 5min. Took the supernatant And detected with Conduct Waters ACQUITY UPLC H-Class, the mobile phase A was 0.1% TFA-$H_2O$, the mobile phase B was 0.1% TFA-ACN, the detection wavelength was 214nm, the column temperature was 30°C, the sample loading volume was 10μl, the flow rate was 0.2ml/min, the running time was 35min, and the running gradient was as follows:

Table 15

| Run time min | Flow ml/min | A% | B% |
|---|---|---|---|
| initial | 0.200 | 90.0 | 10.0 |
| 5.00 | 0.200 | 90.0 | 10.0 |
| 30.00 | 0.200 | 0.0 | 100.0 |
| 32.00 | 0.200 | 0.0 | 100.0 |
| 32.01 | 0.200 | 90.0 | 10.0 |
| 35.00 | 0.200 | 90.0 | 10.0 |

According to the UPLC chromatograph results of the enzymatic reaction mixture, the chromatographic peaks with retention time of 13±0.5min(the peak position of bradykinin) were integrated and summed, and compared with the peak area of bradykinin (1mg/ml, loading volume 10μl) to calculate the concentration of bradykinin generated by the enzymatic reaction. The amount of bradykinin produced by the total reaction system (Table 14) was calculated , and finally converted to the amount of bradykinin(pg/mg) produced per milligram of enzyme at each molar ratio. The results were shown in Table 16.

Table 16

| Molar ratio of substrate to enzyme | Bradykinin production μg/mg(enzyme) | | | |
|---|---|---|---|---|
| | hKLK1X1 | PEG10K-hKLK1X1 | Kb | PEG-Kb |
| **1: 1** | 17.22 | 14.52 | 20.92 | 11.28 |
| **5: 1** | 122.71 | 73.01 | 90.81 | 44.63 |
| **10: 1** | 249.44 | 142.69 | 166.01 | 79.99 |
| **15: 1** | 339.03 | 192.45 | 217.86 | 103.85 |
| **20: 1** | 451.52 | 248.25 | 287.65 | 119.89 |

The detection results based on natural substrates showed that the bradykinin produced by the modified protein was lower than that of the unmodified sample on the whole trend, which was consistent with the characteristics of PEG modified protein. Combined with the data from Examples 9-13, we believed that the PEG-modified protein can more modestly maintain the enzymatic process than the original protein, achieving sustained release of effector molecules. At the same time, the effect of KLK1 is based on the regulation of the KKS system in vivo, which includes the release and clearance of effector molecules. Obviously, a mild and continuous enzymatic process can effectively reduce the clearance mechanism of the KKS system, so that the drugs of the invention can play their biological role more stably

and effectively.

In addition, comparing Kb and hKLK1X1 and their modified products, the sample of the present invention released more effector molecules, which was consistent with the in vivo efficacy results shown in Examples 10-13, further demonstrating that the hKLK1X1 protein of the present invention had a significant advantage over KLK1 of animal origin.

**Example 7 In vitro enzymatic kinetic assay of PEG-modified hKLK1/hKLK1X, etc**

1. Detection method

(1) Standard dilution: The standard pancreatic kallikrein I(from Changzhou Qianhong Biochemical Pharmaceutical) was diluted to 10IU/mL using S2266 substrate buffer (20mM Tris-HCl 8.5), and the diluted standard was diluted to 1, 2, 3, 4, 5, and 6IU/mL according to the table below to make standard curve samples:

| | | | | | | |
|---|---|---|---|---|---|---|
| substrate buffer | 90μL | 80μL | 70μL | 60μL | 50μL | 40μL |
| 10 IU/mL standard | 10μL | 20μL | 30μL | 40μL | 50μL | 60μL |

(2) Substrate with different concentrations: the S2266 substrate was diluted into 400, 200, 100, 50, 25 and 10μM using S2266 substrate buffer.

(3) Sample dilution: Samples were diluted to 1-6 IU/mL using S2266 substrate buffer.

(4) Sample addition: Add the standard in step (1) to the ELISA plate, 80μL/ well, and add each sample to one well. The diluted samples in (3) above were added to the ELISA plate, 80μL/ well of each sample, and 6 wells were added in parallel.

(5) Reading: The microplate reader was set at 37°C and 405nm for kinetic detection, the reading interval was 1min, and the detection time was 15min. When the temperature of the sample cell raised to 37°C, the substrate was added. 80μL/well of 200μM substrate was added to each standard well. Different concentrations of substrate as described in (2) were added to each of the six parallel wells, 80μL/ well. Samples needed to be quickly added and blown and mixed. After the sample loading, the readings should be taken immediately and readings were taken at 1-min intervals.

(7) Results processing: GraphPad prism software was used for results processing, data fitting was performed according to Mie equation, and the enzyme kinetic parameters were calculated. Where Km is Mie constant, which means the concentration of substrate at half of the maximum speed (Vmax) during the enzymatic reaction. Generally, 1/Km is used to approximate the affinity of the enzyme to the substrate. The larger 1/Km means the higher affinity of the enzyme to the substrate , and the enzymatic reaction is easy to proceed.

2. Experimental results

The enzyme kinetics results of each sample were shown in the table below:

Table 17

| Sample | $K_m$ (μmol/L) |
|---|---|
| low glycosylated hKLK1 | 43.76 |
| PEG10K-hKLK1 (low glycosylated) | 79.39 |
| hKLK1X1 | 27.56 |
| PEG5K-hKLK1X1 | 47.23 |
| PEG10K-hKLK1X1 | 46.65 |

According to the results of enzymatic kinetics, the Km value of hKLK1X1 was lower than that of unmutated low glycosylated hKLK1, indicating that the affinity to the substrate increased after the mutation. The Km values of two pegylated mutants were lower than those of pegylated unmutated protein, indicating that two pegylated mutant had better enzymatic properties than pegylated unmutated protein.

**Example 8 Comparison of pharmacokinetics of PEG modified hKLK1 in rats**

1. Grouping

According to the weight of SD rats measured on the first day of the experiment, they were randomly divided into 6 groups: PEG5K-hKLK1(high glycosylated) intravenous injection (0.5mg/kg), PEG5K-hKLK1(high glycosylated) subcutaneous injection (0.5mg/kg), PEG10K-hKLK1(low glycosylated) intravenous injection group (0.5mg/kg), PEG10K-hKLK1(low glycosylated) subcutaneous injection group (0.5mg/kg), PEG10K-hKLK1X1 intramuscular injection (0.1mg/kg), PEG10K-hKLK1X1 intramuscular injection (0.02mg/kg) , 6 rats in each group, half male and half female, staining number. A single dose was administered on the first day of the experiment.

2. Blood sample collection and test sample preparation

Detection time: blood samples were collected at 0min(before drug administration), 30min, 1h, 2h, 4h, 8h, 24h, 2d, 3d, 5d, 7d, a total of 11 times, blood collection volume: 50-100μl serum, for the determination of pharmacokinetic parameters.

Sample preparation:

1) Pharmacokinetics samples: The samples collected were diluted with 10% SD rat mixed blank serum (SD rat mixed blank serum: blocking solution (2% BSA in PBST) = 1:9) to the limit of quantitation (LOQ) concentration range of 2560ng/ml to 80ng/ml.
2) Standard: The PEG modified hKLK1 with high and low glycosylation were diluted with mixed blank serum of SD rats to prepare a series of two-fold gradient concentration standards, 5120ng/ml to 40ng/ml.
3) Quality control: quality control materials with high (1920ng/ml), medium (480ng/ml) and low concentration (240ng/ml) were prepared by diluting PEG modified high and low glycosylated hKLK1 with mixed blank serum of SD rats.

3. pharmacokinetics detection

Drug concentrations in serum were measured by ELISA. The analysis process was as follows:

1) Coating: anti-hKLK1 antibody was diluted to 400ng/well with 20mM phosphate buffer (PB, pH7.4), and the diluted antibody solution was added into the well of ELISA plate (100uL/ well), the plate was sealed, and coated at 4°C overnight. The coated plate was Thermo ELISA plate.
2) Sealing: Discard the liquid in the hole, wash the plate once with a washing machine, 300uL/ hole, and dry. After adding blocking solution (20mM PBS containing 2% BSA and 0.05% Tween20), 200 ul/well, plates were sealed and incubated at 37°C for about 2h.
3) Sample addition: the liquid in the well was removed, and the pharmacokinetics samples, standards and quality control solutions were diluted 10 times with blocking solution, then added to the ELISA plate, 100uL/ well, the plate was sealed, and incubated at 37°C for 1.5h;
4) Adding detection antibody: Discard the liquid in the well, wash the plate with a washing machine for 3 times, 300uL/ well, dry, add the detection antibody working solution (dilute the enzyme-linked anti hKLK1 antibody for detection to 1ug/ml with blocking solution), 100uL/ well, seal the plate, and incubate at 37°C for 45min;
5) Color development: Discard the liquid in the well, wash the plate 5 times with a washing machine, 300uL/well, dry, add TMB No. 1 color development solution, and incubate at 37°C for 15min, depending on the color development situation;
6) Plate reading: 50uL/well of terminating solution (2M $H_2SO_4$) was added to terminate the reaction and the OD value at 450nm was measured immediately. Draw a standard curve with the standard concentration as the X-coordinate and OD value as the Y-coordinate. The sample concentration was calculated.
7) Using Origin 8 software to draw the standard curve and calculate the sample concentration. Microsoft EXCEL was used to calculate the mean, standard deviation and coefficient of variation. GraphPad Prism 7.00 was used to calculate the area under the curve (AUC).

4. Test results

At the same dose, the body exposure (AUC) of the drug was as follows:

Table 18

| Sample | Administration route | AUC (h•ng/ml) | |
|---|---|---|---|
| | | Gender | value |
| PEG5K-hKLK1 (high glycosylated) (0.5mg/kg) | Intravenous injection | Male | 40256 |
| | | Female | 47715 |
| | Subcutaneous injection | Male | 15575 |
| | | Female | 15891 |
| PEG10K-hKLK1 (low glycosylated) (0.5mg/kg) | Intravenous injection | Male | 383645 |
| | | Female | 147389 |
| | Subcutaneous injection | Male | 105376 |
| | | Female | 67261 |
| PEG10K-hKLK1X1 (0.1mg/kg) | Intramuscular injection | Male | 9501 |
| PEG10K-hKLK1X1 (0.02mg/kg) | Intramuscular injection | Male | 2266 |

According to the above pharmacokinetic results, the pharmacokinetic curve of PEG10K-hKLK1(low glycosylated) was significantly better than that of PEG5K-hKLK1(high glycosylated) when injected intravenously and subcutaneously, showing a longer drug half-life. PEG10K-hKLK1X1 still showed better absorption when injected intramuscularly at lower doses.

**Example 9 Comparison of immunogenicity of PEG modified hKLK1 with high and low glycosylation in rats**

1. Grouping

According to the weight of SD rats measured on the first day of the experiment, they were randomly divided into 2 groups: PEG5K-hKLK1(high glycosylated) group and PEG10K-hKLK1(low glycosylated) group, 12 rats in each group, half male and half female, and made a stain number.

2. Administration of drugs

Administration route: intravenous injection.
Administration frequency: once a week for 8 doses.
Administration dose: the concentration of administration was 0.2mg/mL, and the dose was 0.5mg/kg.

3. Blood sample collection

Detection time: blood samples were collected at 0min(before administration), 3 days and 7 days after administration. Blood samples were collected once a week for 2 weeks during the recovery period. About 500μL of whole blood was collected each time, standed for 2 hours, and centrifuged at 3000rpm for 10min. The serum was separated and used for the determination of immunogenicity.

4. Test of immunogenicity

1) Coating: The coating antigen (PEG-modified hKLK1) working solution was added to the ELISA plate, 100uL/ well, and incubated at 2-8°C overnight;
2) Blocking: the liquid in the well was discarded, the plate was washed 3 times, dried, and the blocking solution (20mM PBS containing 2% BSA and 0.05% Tween20) was added to the plate, 200uL/ well, incubated at 37°C for about 2 hours.
3) Sample processing: all animal serum samples were diluted 10-fold with blocking solution;
4) Sample addition: Discard the liquid in the ELISA plate wells, dry, add the samples to each well, 100uL/ well, and

then incubate at 37°C and 200rpm shaking for about 2 hours;

5) Adding the detection antigen working solution: Discard the liquid in the well, wash the plate three times, dry. Add 100uL/ well of detection antigen working solution (dilute biotin-labeled PEG modified hKLK1 to 2.5 μg/ml with blocking solution as the detection antigen working solution)to each well for screening test. In the confirmatory test, 100ul/well of the confirmatory assay antigen working solution was added to each well (PEG modified hKLK1 was diluted to 200μg/ml with the above-mentioned detection antigen working solution as the confirmatory assay antigen working solution), and incubate at 37°C with 200 rpm shaking for about 1 hour.

6) Adding signal amplification detection solution (streptavidin-horseradish peroxidase was diluted to 0.05μg/ml with blocking solution) : Discard the liquid in the well, wash the plate 3 times, then dry, add signal amplification detection solution, 100uL/well, and incubate at 37°C with 200rpm shaking for about 1h;

7) Color development: Discard the liquid in the well, wash the plate 3 times, dry, add color development solution, 100uL/well, and place in the dark at 37°C for 15min;

8) Termination: The reaction was terminated by adding termination solution (2M $H_2SO_4$), 100uL/well, and the OD value at 450nm was immediately measured on a microplate reader.

5. Test results

The immunogenicity evaluation results of Figures 13, 14 showed that anti-drug antibody (ADA) was not detected in the serum of all 12 animals in the PEG-hKLK1(low glycosylated) group, while high ADA values were detected in 2 out of 12 animals in the PEG-hKLK1(high glycosylated) group. This indicated that PEG modified low glycosylated protein was less immunogenic.

**Example 10 In vivo activity comparison of urinary kallikrein for injection and pancreatic kallikrein I for injection**

1. Model preparation

SD rats, male, SPF grade, weighing 270-300g. The cerebral ischemia-reperfusion model of middle cerebral artery occlusion (MCAO) was prepared by using the intraluminal suture method in rats. Animals were anesthetized with gas (isoflurane), and then fixed them in a supine position. The skin was disinfected, and the right common carotid artery, external carotid artery, and internal carotid artery were separated from the midline incision in the neck. The vagus nerve was gently peeled off, and the external carotid artery was ligated and cut. The common carotid artery was clamped near the proximal end, and an incision was made from the distal end of the ligature line of the external carotid artery. The suture(model 2438-A5, purchased from Beijing Xinong Technology Co., LTD.) was inserted and passed through the bifurcation of the common carotid artery into the internal carotid artery. The suture was then slowly inserted until slight resistance was encountered (about 20mm from the bifurcation), blocking the blood supply to the middle cerebral artery. The neck skin was sutured, disinfected, and the rat was returned to the cage. After 90 minutes of ischemia, the rat was anesthetized again, fixed on the rat board, and the neck skin was cut open to find the suture, which was gently removed. Reperfusion was performed, and the neck skin was sutured, disinfected, and the rat was returned to the cage for feeding.

Grouping: There were four groups, namely sham operation group, model group, and pancreatic kallikrein I group for injection (0.4IU/kg, intramuscular injection, pancreatic kallikrein I for injection was PEG10K-modified Kb, the preparation method was as described in Example 5, in which Kb was KLK1 extracted from pig pancreas). Urinary kallikrein group (0.1IU/kg, intravenous injection). In the administration group, the drug was administered 2h after reperfusion. The specific activity of the samples in all animal experiments was calculated according to method 1 of Example 6.

2. Evaluation of neurological defect

The modified Bederson method was used to evaluate neurological defect symptoms.

| |
|---|
| 0: When the tail is suspended, both forelimbs of the animal extend towards the floor without other behavioral deficits. |
| 1: When the tail is suspended, the forelimb of the animal contralateral to the surgery (left) shows wrist and elbow flexion, shoulder internal rotation, elbow abduction, and tight adhesion to the chest wall. |
| 2: When the animal is placed on a smooth plate, the resistance is reduced when the operated side shoulder is moved to the contralateral side. |
| 3: When the animal walks freely, it circles or turns to the opposite side of the surgery. |
| 4: Limb paralysis, no spontaneous movement. |

Measurement of Cerebral Infarction Area

Animals were anesthetized with 10% chloral hydrate, the brain tissue was removed, the olfactory bulbs, cerebellum and lower brainstem were removed, and the bloodstain on brain surface was washed with SPSS. After removing surface residual water, the brain tissue was placed at -80°C for 7 minutes, took out and cut into coronal sections perpendicular to the visual cross plane, and sliced at intervals of 2mm to the posterior direction. The brain slices were placed in fresh TTC (20g/L) dye solution prepared with SPSS at 37°C for 90 minutes. Normal brain tissue was stained deep red, while ischemic brain tissue appeared pale. After washing with SPSS, the brain slices were quickly arranged in sequence from front to back, residual water on the surface was absorbed, and photographed. The image analysis software (Image Tool) was used to delineate the ischemic area (white area) and the right side area on the photos for statistical analysis. The percentage of cerebral infarction area was calculated using the following formula:

$$\text{Cerebral infarction area (\%)} = 100 \times \text{total ischemic area / total right hemisphere area.}$$

4. Results

1). The effect of the test article on the neurological defects symptoms

As shown in FIG. 15 and Table 19, the urinary kallikrein group had a significant improvement in neurological defect symptoms compared with the model group($F_{7,87}$=20.80, P=0.018); Pancreatic kallikrein I for injection significantly improved the neurological defect symptoms compared with the model group($F_{7, 87}$=20.80, P=0.021).

Table 19 Effect of test article on neurological defect symptoms

| Group | Total animals (n) | Dead (n) | Discarded (n) | Samples (n) | Neurological Defect Symptoms Score |
|---|---|---|---|---|---|
| Sham operation group | 8 | 0 | 0 | 8 | 0.00 ± 0.00 |
| Model group | 21 | 9 | 0 | 12 | 2.92 ± 0.15 |
| Urinary kallikrein group | 21 | 8 | 0 | 13 | 1.92 ± 0.21* |
| Pancreatic kallikrein I group | 21 | 8 | 1 | 12 | 1.92 ± 0.15** |
| Mean ± SE. *P<0.05, compared with model group. | | | | | |

2). The effect of the test article on the cerebral infarction area (%)

As shown in Figures 16, 17, 18 and Table 20, urinary kallikrein ($F_{7,87}$=12.72, P=0.042) and pancreatic kallikrein I for injection ($F_{7,87}$=12.72, P=0.019) significantly improved the infarct size compared with the model group.

Table 20 Effect of test article on cerebral infarction area (%)

| Group | Total animals (n) | Dead (n) | Discarded (n) | Samples (n) | Cerebral infarction area (%) |
|---|---|---|---|---|---|
| **Sham operation group** | **8** | **0** | **0** | **8** | **0.00 ± 0.00** |
| **Model group** | **21** | **9** | **0** | **12** | **39.71 ± 2.30** |
| **Urinary kallikrein group** | **21** | **8** | **0** | **13** | **22.74 ± 3.71*** |
| **Pancreatic kallikrein I group** | **21** | **8** | **1** | **12** | **21.05 ± 3.01*** |
| Mean ± SE. *P<0.05, compared with model group. | | | | | |

3). The effect of test article on cumulative mortality rate (%)

As shown in Table 21, animals died in all groups except the sham operation group, and there was no difference between groups in mortality. The urinary kallikrein group ($F_{1,28}$=0.18, P=0.6746), pancreatic kallikrein I for injection group ($F_{1,28}$=0.18, P=0.6753) had no significant difference compared with the model group. 4).

Table 21 Effect of test article on cumulative mortality rate(%)

| Group | D0 | D1 | D5 |
|---|---|---|---|
| **Sham operation group** | 0.00 | 0.00 | 0.00 |
| **Model group** | 0.00 | 23.81 | 42.86 |
| **Urinary kallikrein group** | 0.00 | 23.81 | 38.10 |
| **pancreatic kallikrein I group** | 0.00 | 23.81 | 38.10 |

Conclusion

Compared with the model group, urinary kallikrein and pancreatic kallikrein I for injection had significant protective effect on the brain after ischemia-reperfusion.

**Example 11 In vivo activity evaluation of PEG10K modified unmutated hyperglycosylated hKLK1**

1. Model preparation, evaluation of neurological defect symptoms, and measurement of cerebral infarction area were the same as in Example 10.

Grouping and administration: There were 4 groups, namely sham operation group, model group, positive drug group (pancreatic kallikretin I for injection, 0.4IU/kg, intramuscular injection), PEG10K-hKLK1(high glycosylated) group (0.1IU/kg, intravenous injection), drug was given 2 hours after reperfusion.

2. Results

1) Effect of the test article on the neurological defect symptoms

As shown in FIG. 19 and Table 22, PEG10K-hKLK1(high glycosylated) group ($F_{3,43}$=63.32,P=0.352) had a certain improvement in neurological defect symptoms compared with the model group, but the difference was not statistically significant. Compared with the model group, the positive drug group ($F_{3,44}$=56.34, P=0.006) had a significant improvement in neurological defect symptoms.

Table 22 Effect of test article on neurological defect symptoms

| Group | Total animals (n) | Dead (n) | Discarded (n) | Samples (n) | Neurological Defect Symptoms Score |
|---|---|---|---|---|---|
| Sham operation group | 8 | 0 | 0 | 8 | 0.00 ± 0.00 |
| Model group | 21 | 8 | 0 | 13 | 3.15 ± 0.15 |
| Positive drug group | 21 | 7 | 1 | 13 | 2.23 ± 0.17* |
| PEG10K-hKLK1 (high glycosylated) group | 21 | 7 | 1 | 13 | 2.77 ± 0.17 |
| Mean ± SE. *P<0.05, compared with model group. | | | | | |

2) The effect of the test article on the cerebral infarction area (%)

As shown in Figures 20, 21, 22 and Table 23, PEG10K-hKLK1(high glycosylated) group ($F_{3,43}$=37.72, P=0.197) had a certain improvement in cerebral infarction area compared with the model group, but the difference was not significant; Compared with the model group, the positive drug group ($F_{3,44}$=34.58, P = 0.007) had a significant improvement in cerebral infarction area.

Table 23 Effect of test article on cerebral infarction area (%)

| Group | Total animals (n) | Dead (n) | Discarded (n) | Samples (n) | Cerebral infarction area (%) |
|---|---|---|---|---|---|
| Sham operation group | 8 | 0 | 0 | 8 | 0.00 ± 0.00 |
| Model group | 21 | 8 | 0 | 13 | 44.76 ± 2.46 |
| Positive drug group | 21 | 7 | 1 | 13 | 29.42 ± 2.91* |
| PEG10K-hKLK1 (high glycosylated) group | 21 | 7 | 1 | 13 | 36.40± 3.35 |
| Mean ± SE. *P<0.05, compared with model group. | | | | | |

3) The effect of test article on cumulative mortality rate (%)

As shown in Table 24 below, animals died in all groups except the sham operation group, and there was no difference between groups in mortality. There was no significant difference between positive drug group ($F_{1,6}$=0.00,P=1.0000), PEG10K-hKLK1(high glycosylated) group ($F_{1,6}$=0.00,P =1.0000) and model group.

Table 24 Effect of test article on cumulative mortality rate (%)

| Group | D1 | D2 |
|---|---|---|
| Sham operation group | 0.0 | 0.0 |
| Model group | 17.39 | 34.78 |
| Positive drug group | 21.74 | 30.43 |
| PEG10K-hKLK1 (high glycosylated) group | 21.74 | 30.43 |

4) Conclusion

When animals were treated with the test article after ischemia-reperfusion, PEG10K-hKLK1(high glycosylated) only tended to improve symptoms compared with the model group, but the difference was not statistically significant.

**Example 12 In vivo activity evaluation of different PEG modification products of unmutated low glycosylated hKLK1**

1. Model preparation, evaluation of neurological defect symptoms, and measurement of cerebral infarction area were the same as in example 10.

Grouping and administration: There were 7 groups, sham operation group, model group and positive drug group (pancreatic kallikrein I for injection, 0.4IU/kg, intramuscular injection), PEG40K-hKLK1(low glycosylated) group (0.1IU/kg), PEG30K-hKLK1(low glycosylated) group (0.1IU/kg), PEG10K-hKLK1(low glycosylated) group (0. HU/kg), PEG5K-hKLK1(low glycosylated) group (0.1IU/kg). The drug was injected intravenously 2h after reperfusion in the administration group. The dose of PEG-hKLK1 was converted to the specific activity, and the animals were administrated with the same activity unit of PEG-hKLK1 per kilogram.

2. Results

1) The effect of the test article on the neurological defect symptoms

As shown in Figure 23 and Table 25 below, PEG40K-hKLK1(low glycosylated) group ($F_{3,44}$=46.28,P=0.209), PEG30K-hKLK1(low glycosylated) group ($F_{3,43}$=53.33,P=0.209), PEG5K-hKLK1(low glycosylated) group ($F_{3,44}$=46.28, P=0.209) had a certain improvement in neurological defect compared with the model group, but the difference was not statistically significant. Compared with the model group, PEG10K-hKLK1(low glycosylated) group ($F_{3,45}$=46.43, P =0.008) and positive drug group ($F_{3,45}$=46.43, P=0.003) had a significant improvement in neurological defect.

Table 25 Effect of test article on neurological defect symptoms

| Group | Total animals (n) | Dead (n) | Discarded (n) | Samples (n) | Neurological Defect Symptoms Score |
|---|---|---|---|---|---|
| Sham operation group | 8 | 0 | 0 | 8 | 0.00 ± 0.00 |
| Model group | 21 | 8 | 0 | 13 | 3.08 ± 0.14 |
| Positive drug group | 21 | 7 | 1 | 13 | 2.15 ± 0.22* |
| PEG40K-hKLK1 (low glycosylated) group | 21 | 7 | 0 | 14 | 2.57 ± 0.17 |
| PEG30K-hKLK1 (low glycosylated) group | 21 | 8 | 0 | 13 | 2.62 ± 0.14 |
| PEG10K-hKLK1 (low glycosylated) group | 21 | 6 | 0 | 15 | 2.27 ± 0.15* |
| PEG5K-hKLK1 (low glycosylated) group | 21 | 7 | 0 | 14 | 2.57 ± 0.17 |
| Mean ± SE. *P<0.05, compared with model group. | | | | | |

2) The effect of the test article on the cerebral infarction area (%)

As shown in Figures 24, 25, 26 and Table 26, PEG40K-hKLK1(low glycosylated) group ($F_{3,44}$=37.73, P=0.446), PEG30K-hKLK1(low glycosylated) group ($F_{3,43}$=36.56, P=0.502), PEG5K-hKLK1(low glycosylated) group ($F_{3,44}$=39.05,P=0.218) had a certain improvement in cerebral infarction area compared with the model group, but there was no significant difference. Compared with the model group, the PEG10K-hKLK1(low glycosylated) group ($F_{3,45}$=39.30,P=0.001) and the positive drug group ($F_{3,45}$=39.30,P=0.001) had a significant improvement in cerebral infarction area.

Table 26 Effect of test article on cerebral infarction area (%)

| Group | Total animals (n) | Dead (n) | Discarded (n) | Samples (n) | Cerebral infarction area (%) |
|---|---|---|---|---|---|
| Sham operation group | 8 | 0 | 0 | 8 | 0.00 ± 0.00 |
| Model group | 21 | 8 | 0 | 13 | 42.41 ± 2.26 |
| Positive drug group | 21 | 7 | 1 | 13 | 27.36 ± 3.29* |
| PEG40K-hKLK1 (low glycosylated) group | 21 | 7 | 0 | 14 | 36.48 ± 2.72 |
| PEG30K-hKLK1 (low glycosylated) group | 21 | 8 | 0 | 13 | 36.66 ± 2.97 |
| PEG10K-hKLK1 (low glycosylated) group | 21 | 6 | 0 | 15 | 27.72 ± 2.09* |
| PEG5K-hKLK1 (low glycosylated) group | 21 | 7 | 0 | 14 | 34.92 ± 2.47 |
| Mean ± SE. *P<0.05, compared with model group. | | | | | |

3) Effect of test article on cumulative mortality rate (%)

As shown in Table 27, animals died in all groups except the sham operation group, and there was no difference between groups in mortality. Compared with the model group, there was no statistical differences between positive drug group ($F_{1,3}$=0.15, P=0.7239), PEG40K-hKLK1(low glycosylated) group ($F_{1,3}$=0.17, P=0.7100), PEG30K-hKLK1(low glycosylated) group ($F_{1,3}$=0.17, P=0.7106), PEG10K-hKLK1(low glycosylated) group ($F_{1,3}$=0.15, P=0.7239), PEG5K-hKLK1(low glycosylatied) group ($F_{1,3}$=0.00, P=0.9994) .

Table 27 Effect of test article cumulative mortality rate (%)

| Group | D1 | D2 |
|---|---|---|
| Sham operation group | 0.00 | 0.00 |

(continued)

| Group | D1 | D2 |
|---|---|---|
| Model group | 19.05 | 38.10 |
| Positive drug group | 19.05 | 33.33 |
| PEG40K-hKLK1 (low glycosylated) group | 19.05 | 33.33 |
| PEG30K-hKLK1 (low glycosylated) group | 23.81 | 38.10 |
| PEG10K-hKLK1 (low glycosylated) group | 23.81 | 28.57 |
| PEG5K-hKLK1 (low glycosylated) group | 23.81 | 33.33 |

4) Conclusion

The results showed that: for unmutated hKLK1 with low glycosylation, PEG10K-hKLK1(low glycosylated) showed significant brain protective efficacy in animal models, and PEG5K-hKLK1(low glycosylated), PEG40K-hKLK1(low glycosylated), and PEG30K-hKLK1(low glycosylated) all showed a trend of improving symptoms. At the same time, the in vivo efficacy results were also consistent with the in vitro efficacy. The in vitro activity of PEG10K modified low glycosylated hKLK1 was higher than that of PEG-5K modified low glycosylated hKLK1, and higher than PEG30K/40K modified low glycosylated hKLK1.

Combined with the results of examples 11 and 12, PEG10K-hKLK1(high glycosylated) is less effective than PEG10K-hKLK1(low glycosylated) with the same dosage. The results showed that the unmutated low glycosylated samples were better than the high glycosylated samples with the same PEG modification.

**Example 13 In vivo activity evaluation of different PEG modified hKLK1 mutants**

1. Model preparation, evaluation of neurological defect symptoms, and measurement of cerebral infarction area were the same as in Example 10.

Grouping: there were 4 groups , namely sham operation group, model group, PEG5K-hKLK1X1 group (0.1IU/kg), PEG10K-hKLK1X1 group (0.1IU/kg). The animals were injected intravenously 2h after reperfusion.

2. The results

1) The effect of the test article on neurological defect symptoms

As shown in Figure 27 and Table 28, the PEG5K-hKLK1X1 group ($F_{2,38}$=67.13, P=0.017) had a significant improvement in neurological defect compared with the model group; The PEG10K-hKLK1X1 group ($F_{2,37}$=86.65, P=0.003) had a extremely significant improvement in neurological defect compared with the model group.

Table 28 Effect of test article on neurological defect symptoms

| Group | Total animals (n) | Dead (n) | Discarded (n) | Samples (n) | Neurological Defect Symptoms Score |
|---|---|---|---|---|---|
| Sham operation group | 8 | 0 | 0 | 8 | 0.00 ± 0.00 |
| Model group | 18 | 2 | 0 | 16 | 2.69 ± 0.12 |
| PEG5K-hKLK1X1 group | 18 | 1 | 0 | 17 | 2.12 ± 0.17* |
| PEG10K-hKLK1X1 group | 18 | 2 | 0 | 16 | 2.06 ± 0.14** |
| Mean ± SE. *P<0.05, **P<0.01, compared with model group. | | | | | |

2) The effect of the test article on the cerebral infarction area (%)

As shown in Figures 28, 29, 30 and Table 29, the PEG5K-hKLK1X1 group ($F_{2,38}$=39.50, P=0.001) and PEG10K-hKLK1X1 group ($F_{2,37}$=90.50, P=0.000) had a significant improvement in cerebral infarction area compared with the model group.

Table 29 Effect of test article on cerebral infarction area (%)

| Group | Total animals (n) | Dead (n) | Discarded (n) | Samples (n) | Cerebral infarction area (%) |
|---|---|---|---|---|---|
| Sham operation group | 8 | 0 | 0 | 8 | 0.00 ± 0.00 |
| Model group | 18 | 2 | 0 | 16 | 38.73 ± 1.99 |
| PEG5K-hKLK1X1 group | 18 | 1 | 0 | 17 | 23.60 ± 3.28** |
| PEG10K-hKLK1X1 group | 18 | 2 | 0 | 16 | 22.92 ± 1.71*** |
| Mean ± SE. *P<0.05, **P<0.01, ***P<0.001 compared with the model group. | | | | | |

3) Effect of test article on cumulative mortality rate (%)

As shown in Table 30, animals died in all groups except the sham operation group, and there was no difference between groups in mortality. There was no significant difference between the PEG5K-hKLK1X1 group ($F_{1,5}$=0.71, P=0.4369) and the PEG10K-hKLK1X1 group ($F_{1,5}$=0.00, P=1.0000) with the model group.

Table 30 Effect of test article on cumulative mortality rate (%)

| Group | D0 | D1 |
|---|---|---|
| Sham operation group | 0.0 | 0.0 |
| Model group | 0.00 | 11.11 |
| PEG5K-hKLK1X1 group | 0.00 | 5.56 |
| PEG10K-hKLK1X1 group | 0.00 | 11.11 |

4) Conclusion

Compared with the model group, PEG5K-hKLK1X1 and PEG10K-hKLK1X1 showed significant brain protection after ischemia-reperfusion.

In combination with examples 12 and 13, the in vivo efficacy results of PEG-hKLK1(unmutated)/hKLK1X(mutant) indicated that:

Table 31

| Protein | In vivo efficacy after PEG-5K modification | In vivo efficacy after PEG-10K modification |
|---|---|---|
| low glycosylated hKLK1 (unmutated) | trend of improvement in neurological defect, with no statistical difference; improvement in the cerebral infarction area, with no statistical difference | significant therapeutic effect on neurological defect (*$P < 0.05$); significant improvement in the cerebral infarction area (*$P < 0.05$) |
| hKLK1X (mutant) | significant therapeutic effect on neurological defect (*$P < 0.05$); extremely significant improvement in the cerebral infarction area (*$P < 0.01$) | extremely significant therapeutic effect on neurological defect (**$P < 0.01$); very significant improvement in the cerebral infarction area (*$P < 0.001$) |

The proteins with N141 mutation were modified with the same PEG. The PEG-modified protein with mutation had better efficacy, better performance, and better therapeutic effect in vivo that the PEG-modified protein without mutation.

Results of in vivo effects of drugs in examples 10-13:

Table 32

| Sample | Dose IU/kg | In vivo effect |
|---|---|---|
| urinary kallikrein | 0.1 | significant therapeutic effect on neurological defect (* $P < 0.05$)<br>significant improvement in the cerebral infarction area (* $P < 0.05$) |
| pancreatic kallikrein I | 0.4 | significant therapeutic effect on neurological defect (* $P < 0.05$);<br>significant improvement in the cerebral infarction area (* $P < 0.05$) |
| PEG10K-hKLK1 (low glycosylated) | 0.1 | significant therapeutic effect on neurological defect (* $P < 0.05$);<br>significant improvement in the cerebral infarction area (* $P < 0.05$) |
| PEG10K-hKLK1 (high glycosylated) | 0.1 | trend of improvement in neurological defect, with no statistical difference;<br>trend of improvement in the cerebral infarction area,with no statistical difference |
| PEG5K-hKLK1X1 | 0.1 | significant therapeutic effect on neurological defect (* $P < 0.05$);<br>extremely significant improvement in the cerebral infarction area (* $P < 0.01$) |
| PEG10K-hKLK1X1 | 0.1 | extremely significant therapeutic effect on neurological defect (** $P < 0.01$);<br>very significant improvement in the cerebral infarction area (* $P < 0.001$) |
| * Pancreatic kallikrein I for injection was administered intramuscularly, and the other samples were administered intravenously. | | |

With the same polyethylene glycol modification, the efficacy of unmutated hKLK1 with low glycosylation was better than that of unmutated hKLK1 with high glycosylation; the hKLK1 protein with mutation at NFS had better efficacy than the unmutated low glycosylated hKLK1, and had obvious advantages at low doses.

**Example 14 Pharmacodynamic study of PEG-hKLK1X1 with different administration methods**

1. Model preparation, evaluation of neurological defect symptoms, and measurement of cerebral infarction area were the same as in Example 10.

Grouping: There were three groups, sham operation group, model group and PEG10K-hKLK1X1 (intramuscular injection) group(0.4IU/kg). The animals were administered 2h after reperfusion.

2. Results

1)The effect of the test article on the neurological defects symptoms

As shown in FIG. 31 and Table 33, the PEG10K-hKLK1X1 intramuscular injection group ($F_{2,36}$=122.84, P=0.000) had a very significant improvement in neurological defect symptoms compared with the model group.

Table 33 Effect of test article on neurological defect symptoms

| Group | Total animals (n) | Dead (n) | Discarded (n) | Samples (n) | Neurological Defect Symptoms Score |
|---|---|---|---|---|---|
| Sham operation group | 8 | 0 | 0 | 8 | 0.00 ± 0.00 |
| Model group | 20 | 5 | 0 | 15 | 3.00 ± 0.10 |
| PEG10K-hKLK1X1 group | 20 | 4 | 0 | 16 | 2.25 ± 0.14*** |
| Mean ± SE. *P<0.05, **P<0.01, ***P<0.001, compared with the model group. | | | | | |

2) The effect of the test article on the area of cerebral infarction (%)

As shown in Figure 32 and Table 34 below, PEG10K-hKLK1X1 intramuscular injection ($F_{2,36}$=55.02, P=0.001) had extremely significant improvement in cerebral infarct size compared with the model group.

Table 34 Effect of test article on cerebral infarction area (%)

| Group | Total animals (n) | Dead (n) | Discarded (n) | Samples (n) | Cerebral infarction area (%) |
|---|---|---|---|---|---|
| Sham operation group | 8 | 0 | 0 | 8 | 0.00 ± 0.00 |
| Model group | 20 | 5 | 0 | 15 | 38.79 ± 2.01 |
| PEG10K-hKLK1X1 group | 20 | 4 | 0 | 16 | 25.93 ± 2.68** |
| Mean ± SE. *P<0.05, **P<0.01, compared with model group. | | | | | |

3) Conclusion

In combination with examples 13 and 14, PEG10K-hKLK1X1 by intravenous injection (0.1IU/kg) and intramuscular injection (0.4IU/kg) all had a very significant brain protection effect, and can effectively treat the neurological defect symptoms and improve the cerebral infarction area in animals after stroke.

In summary, the low glycosylated KLK1 had higher activity than the high glycosylated KLK1; Compared with the unmutated KLK1, the recombinant KLK1 mutant lacked N-glycosylation, and the purification process was simpler, the product was more homogeneous, the quality was more controllable, and the yield was higher. Moreover, KLK1 mutant had further advantages in enzymatic properties and activity compared with the unmutated low glycosylated KLK1. In addition, the pegylated recombinant hKLK1 in the present application had significant efficacy in a variety of administration methods, and had the advantages of safety and long-term effect of pegylated drug, which can reduce the administration frequency and improve patient compliance. This application covered the prevention, treatment, prognosis recovery, and prevention of recurrence of acute ischemic stroke, peripheral neuropathy, retinopathy, ocular fundus disease, hypertension, diabetic nephropathy, IgA nephritis, chronic kidney disease and other diseases.

## Claims

1. Low glycosylated kallikrein I or its derivative, which is primate kallikrein I containing three N-glycosylation sites of native kallikrein I at NMS, NHT and NFS, of which asparagine at NFS has no glycosylation or a small amount of glycosylation, "low glycosylated" or "small amount of glycosylation" means that the proportion of glycosylated asparagine at NFS is ≤ 10%, < 9%, < 8%, < 7%, < 6%, < 5%, < 4%, < 3%, < 2%, < 1%, < 0.5% or ≤ 0.1%.

2. Recombinant kallikrein I mutant or its derivative, which is primate kallikrein I with only two N-glycosylation sites.

3. The recombinant kallikrein I mutant or its derivative of claim 2, which retains the N-glycosylation at NMS and NHT of the native kallikrein I and does not contain the N-glycosylation at NFS of the native kallikrein I

4. The recombinant kalinolysin I mutant or its derivative of claim 2, wherein the asparagine at NFS of the kalinolysin I is mutated to any other amino acid except asparagine, and 0, 1 or 2 amino acids of F and S at NFS are mutated to any other amino acid.

5. The recombinant kallikrein I mutant or its derivative of claim 2, wherein the phenylalanine at NFS of the kallikrein I is mutated to proline, and 0, 1, or 2 amino acids of N and S at NFS are mutated to any other amino acid.

6. The recombinant kalinolysin I mutant or its derivative of claim 2, wherein the serine at NFS of the kalinolysin I is mutated to any other amino acid except serine and threonine, and 0, 1 or 2 amino acids of N and F at NFS are mutated to any other amino acid.

7. The recombinant kallikrein I mutant or its derivative of claim 4, wherein the asparagine at the NFS of the kallikrein I is mutated to neutral polar amino acid, acidic amino acid, basic amino acid or aliphatic amino acid.

**8.** The recombinant kallikrein I mutant or its derivative of claim 7, wherein the asparagine at the NFS of the kallikrein I is mutated to glutamine (Gln), aspartic acid (Asp), arginine (Arg) or alanine (Ala).

**9.** The recombinant kallikrein I mutant or its derivative of any one of claims 2 to 8, wherein said kallikrein I is human kallikrein I, the asparagine, phenylalanine and serine at NFS are the 141st, 142nd, and 142rd amino acid of human kallikrein I respectively; the amino acid sequences of native human kallikrein I are shown in Genbank accession numbers AAA59455.1, NP002248.1, AAA36136.1, AAP35917, or AAU12569.

**10.** The recombinant kallikrein I mutant or its derivative of any one of claims 2 to 8, amino acid sequence of said mutant is shown as SEQ ID No: 3, SEQ ID No: 4, SEQ ID No: 5 or SEQ ID No: 6.

**11.** A composition containing kallikrein I or its derivative of claim 1, or recombinant kallikrein I mutant or its derivative of any one of claims 2-10.

**12.** Application of Kallikrein I or its derivative of claim 1, or recombinant kallikrein I mutants or its derivative of any one of claims 2-10 in the preparation of drugs for the treatment, prevention, recovery, and prevention of recurrence of acute ischemic stroke, peripheral neuropathy, retinopathy, fundus disease, hypertension, diabetic nephropathy, IgA nephritis, and chronic kidney disease.

**13.** Pegylated kallikrein I, said kallikrein I is modified by polyethylene glycol modifier, kallikrein I is the kallikrein I of claim 1 or recombinant kallikrein I mutant of any one of claims 2-10.

**14.** The pegylated kallikrein I of claim 13, the PEG modifier is straight chain PEG succinimidyl propionate with molecular weight of 5kDa-10kDa, the general formula is as shown in (1),

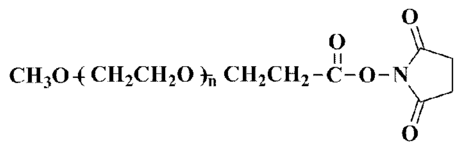

(1),

where n is an integer from 105 to 225.

**15.** The pegylated kallikretin I of claim 13, the pegylated modifier is branched polyethylene glycol propionaldehyde with molecular weight of 30kDa-40kDa, the general formula is shown in (2),

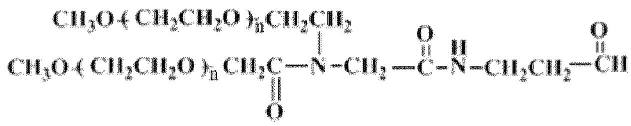

(2),

where n is an integer from 335 to 455.

**16.** A composition containing pegylated kallikrein I of any one of claims 13 to 15.

**17.** Application of pegylated kallikrein I of any one of claims 13-15 in the preparation of drugs for the treatment, prevention, recovery, and prevention of recurrence of acute ischemic stroke, peripheral neuropathy, retinopathy, fundus disease, hypertension, diabetic nephropathy, IgA nephritis, and chronic kidney disease.

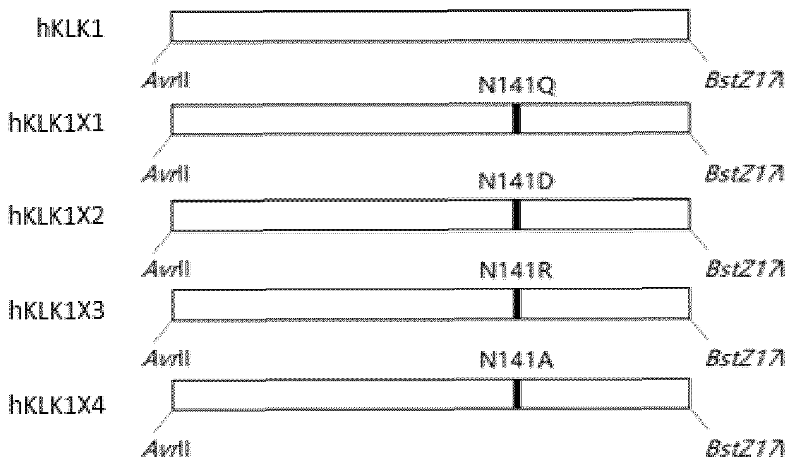


Figure 1

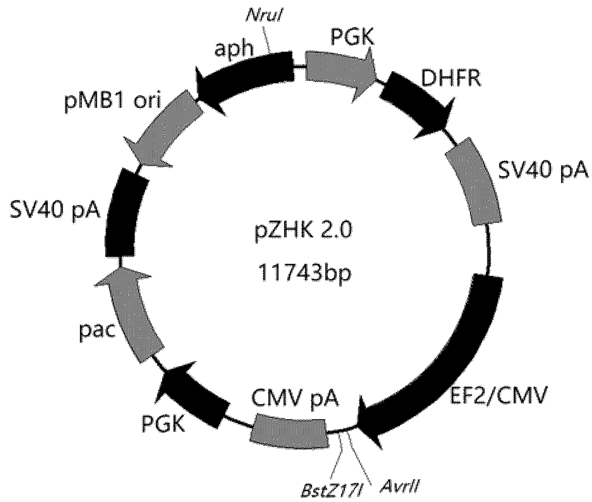


Figure 2

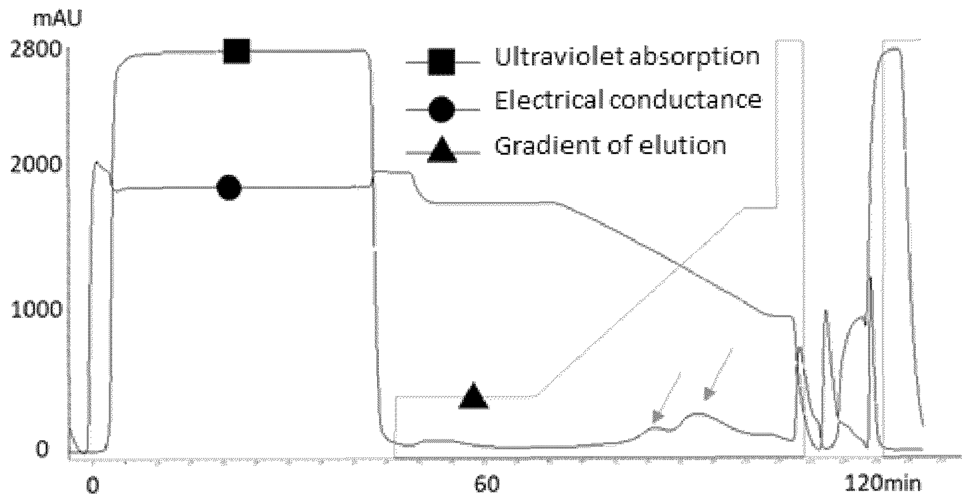


Figure 3

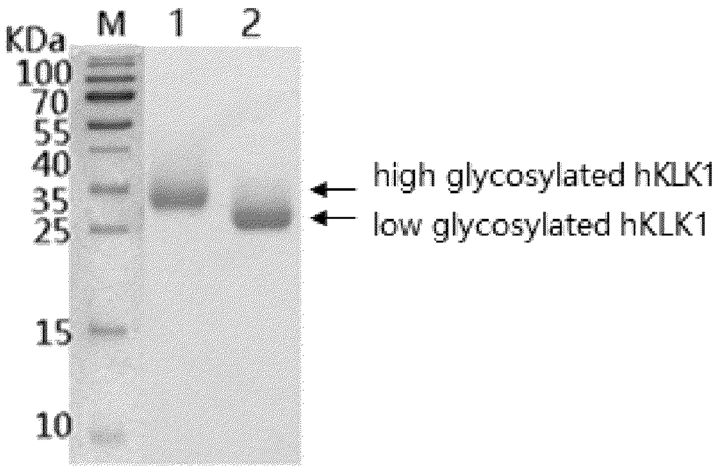


Figure 4

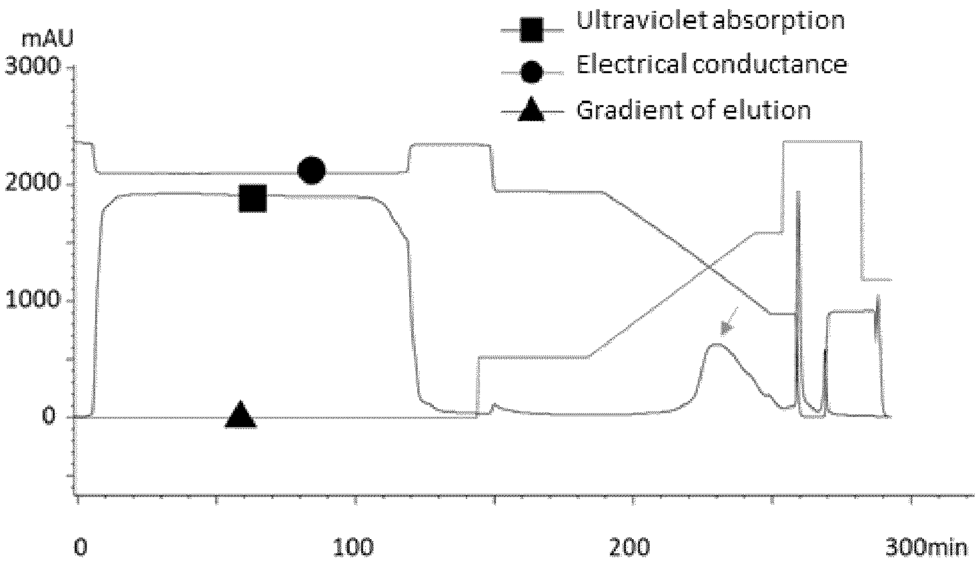


Figure 5

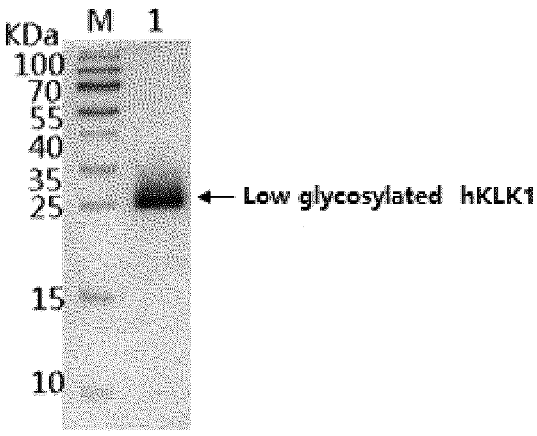


Figure 6

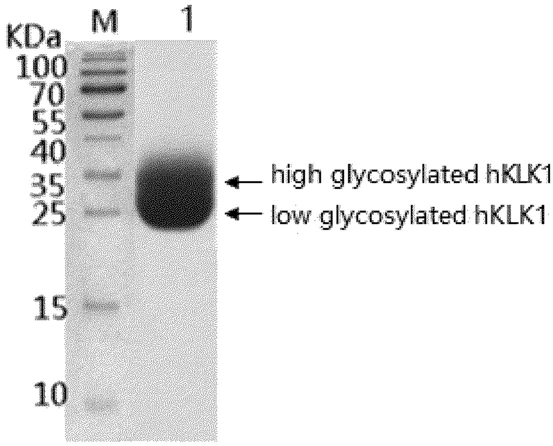


Figure 7

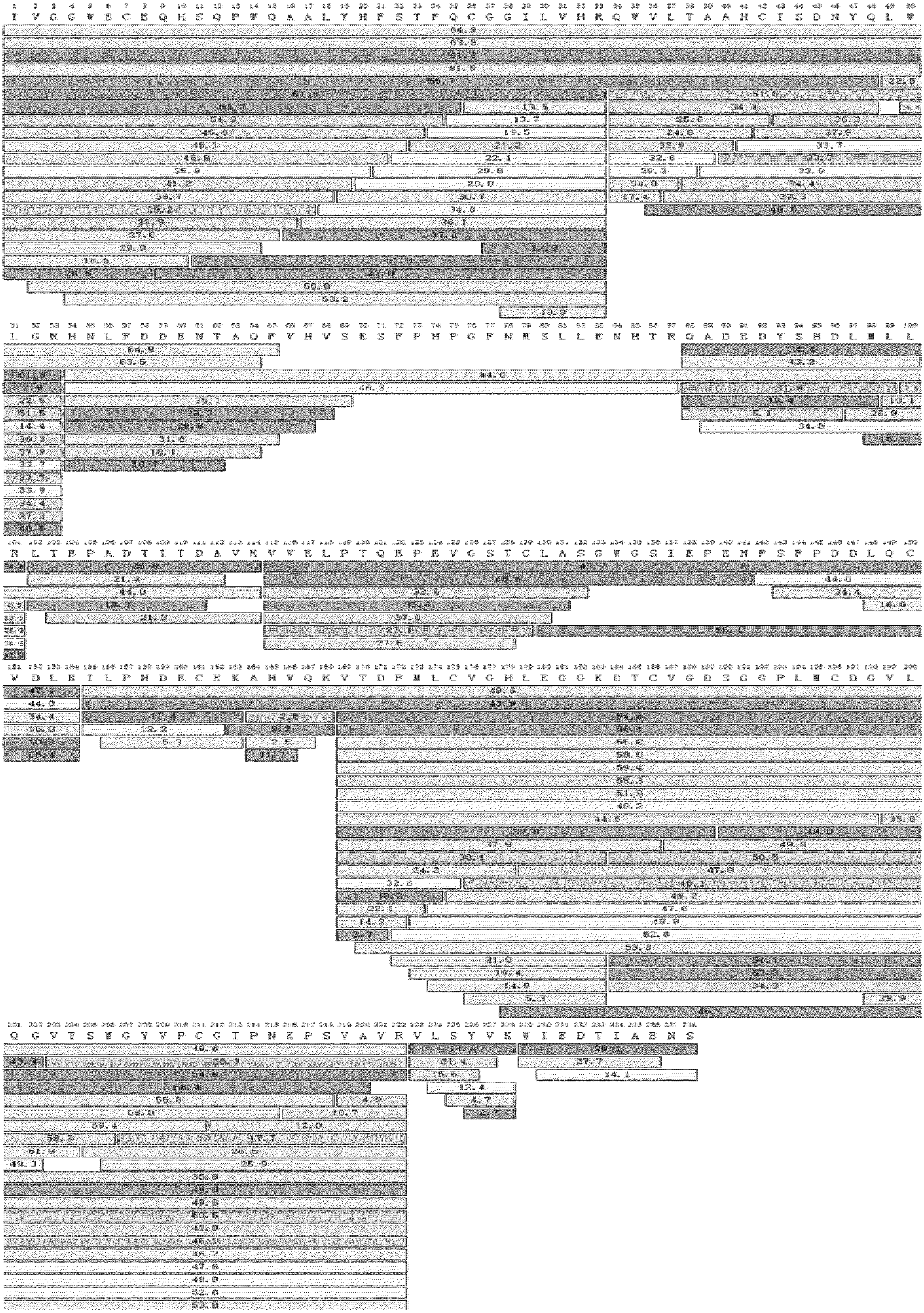

Figure 8

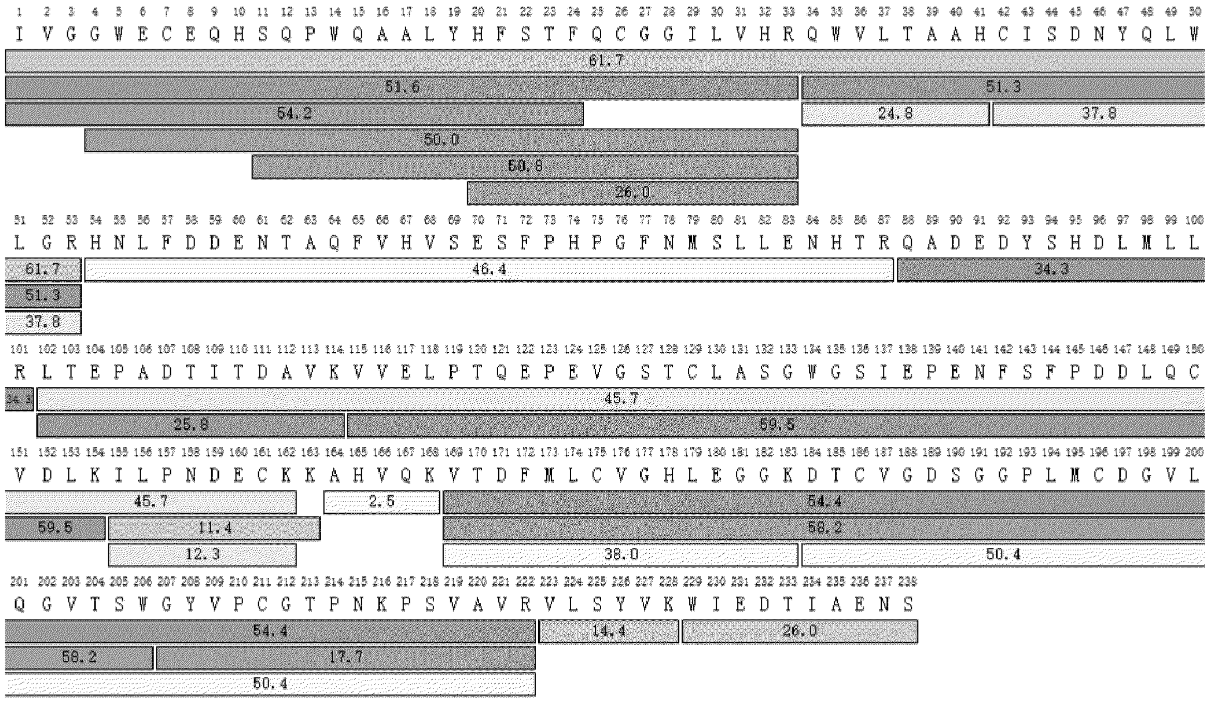


Figure 9

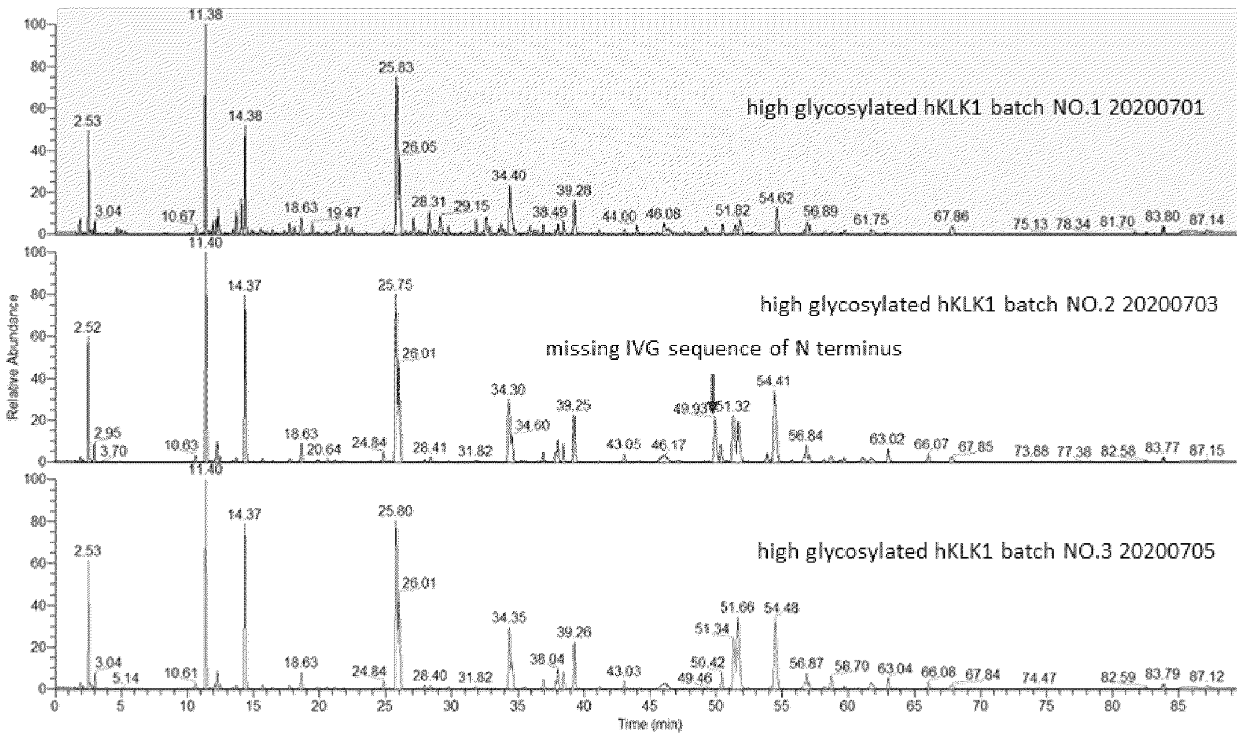


Figure 10

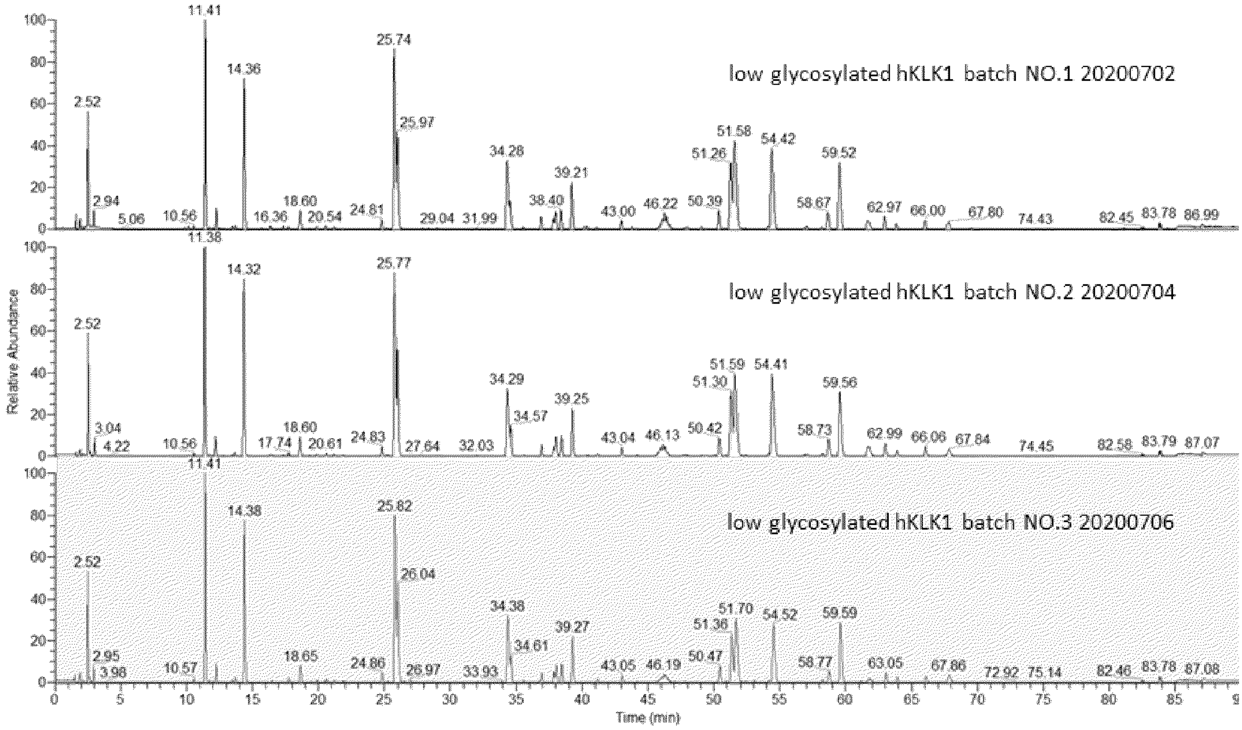
low glycosylated hKLK1 batch NO.1 20200702
low glycosylated hKLK1 batch NO.2 20200704
low glycosylated hKLK1 batch NO.3 20200706
Relative Abundance
Time (min)

Figure 11

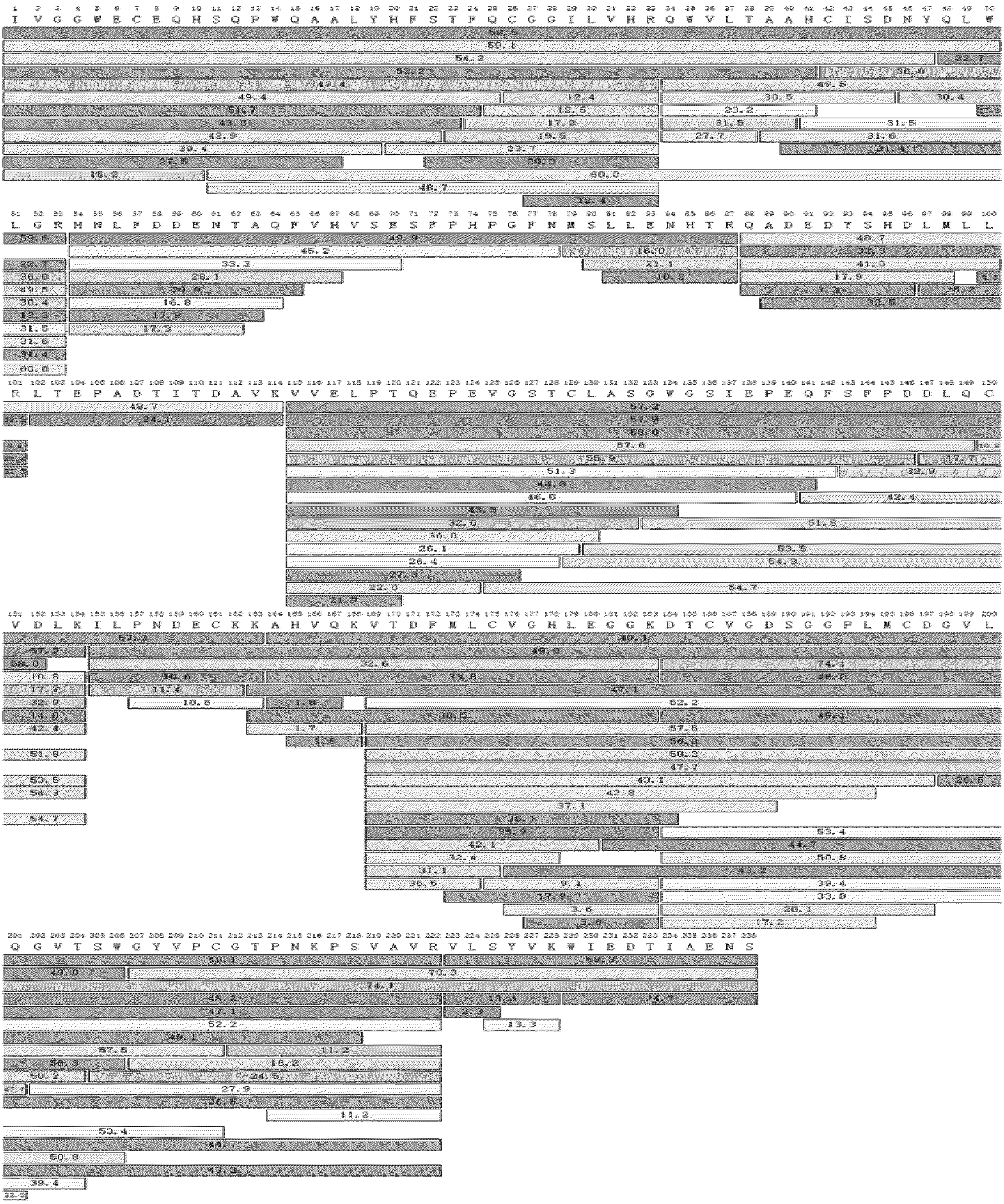

Figure 12

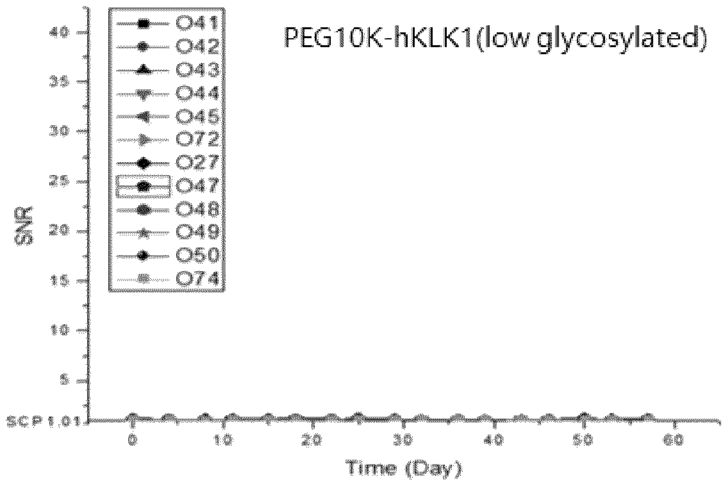


Figure 13

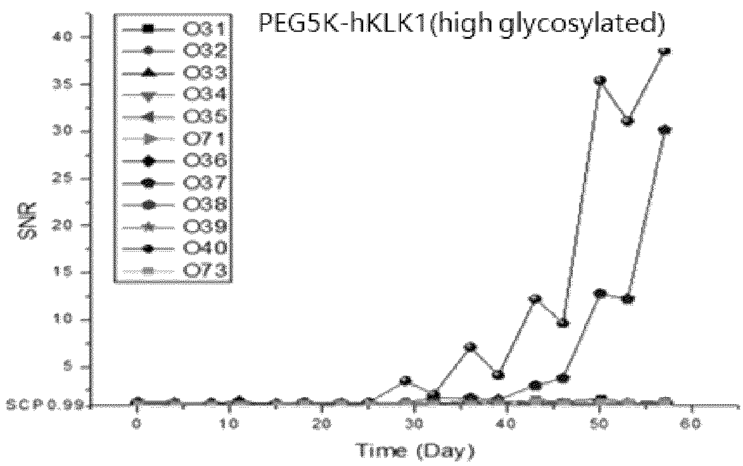


Figure 14

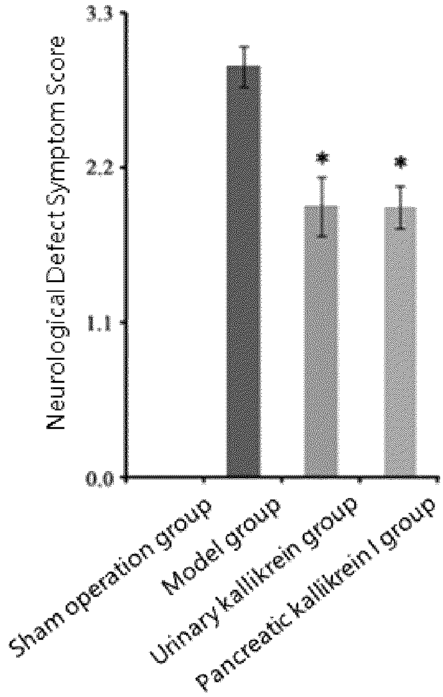


Figure 15

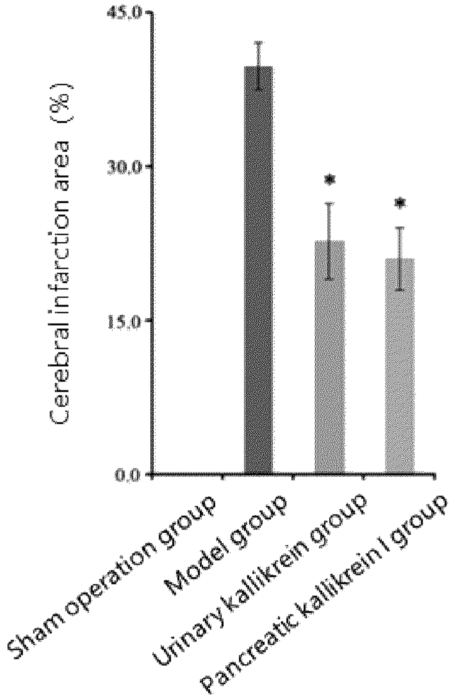


Figure 16

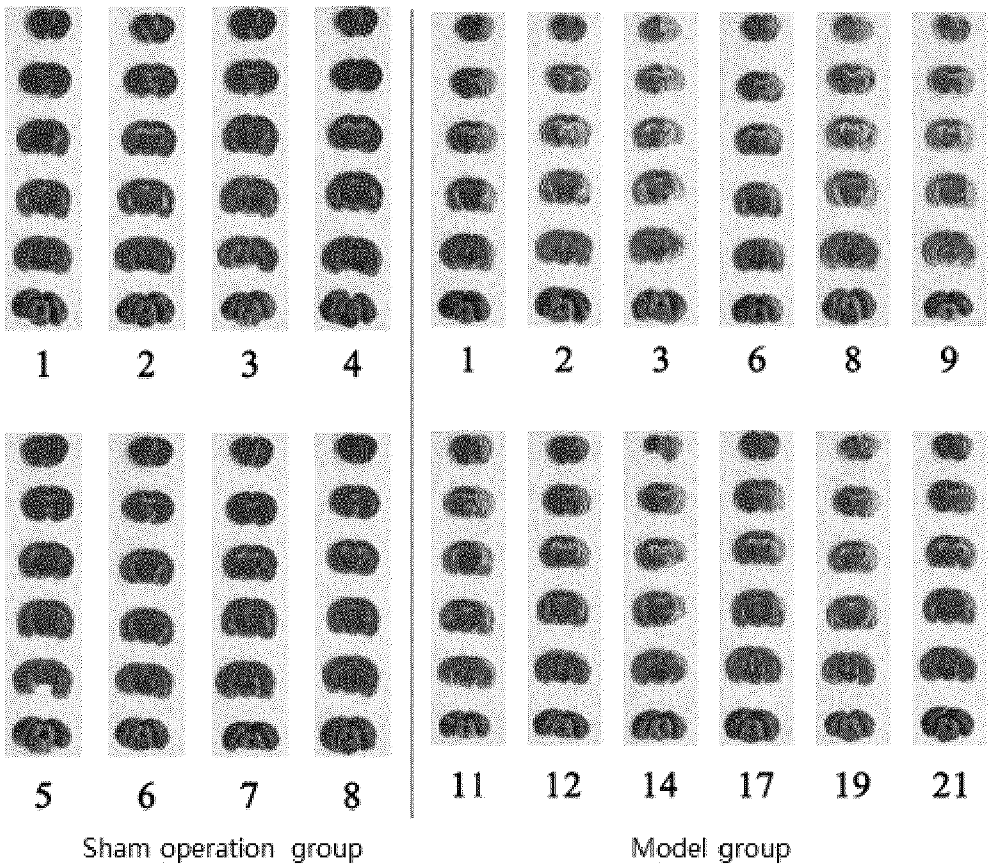


Figure 17

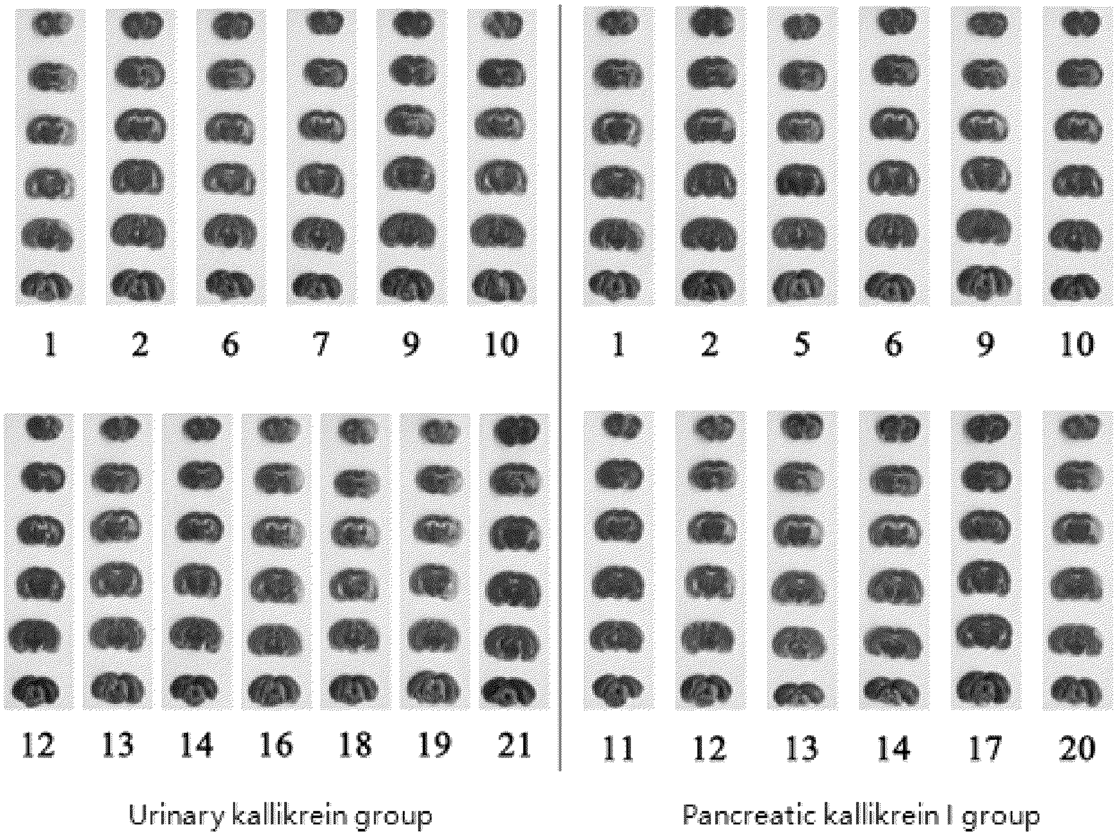


Figure 18

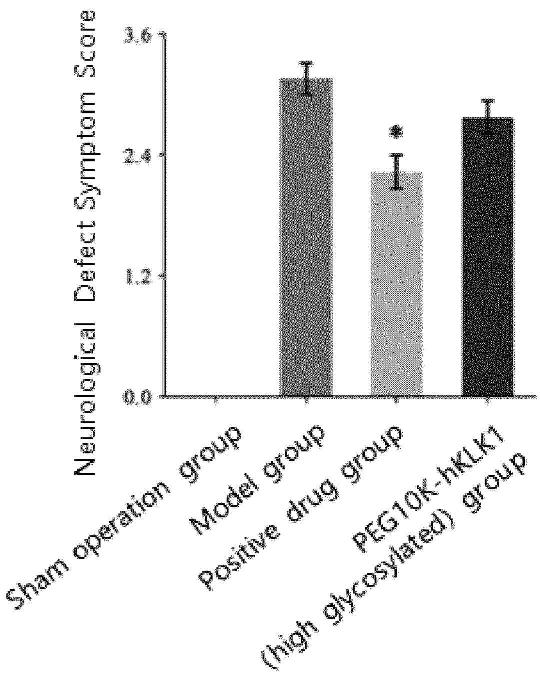


Figure 19

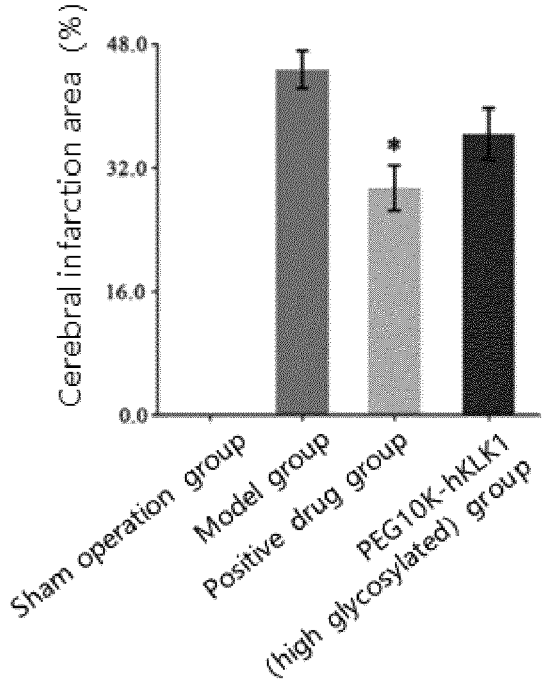


Figure 20

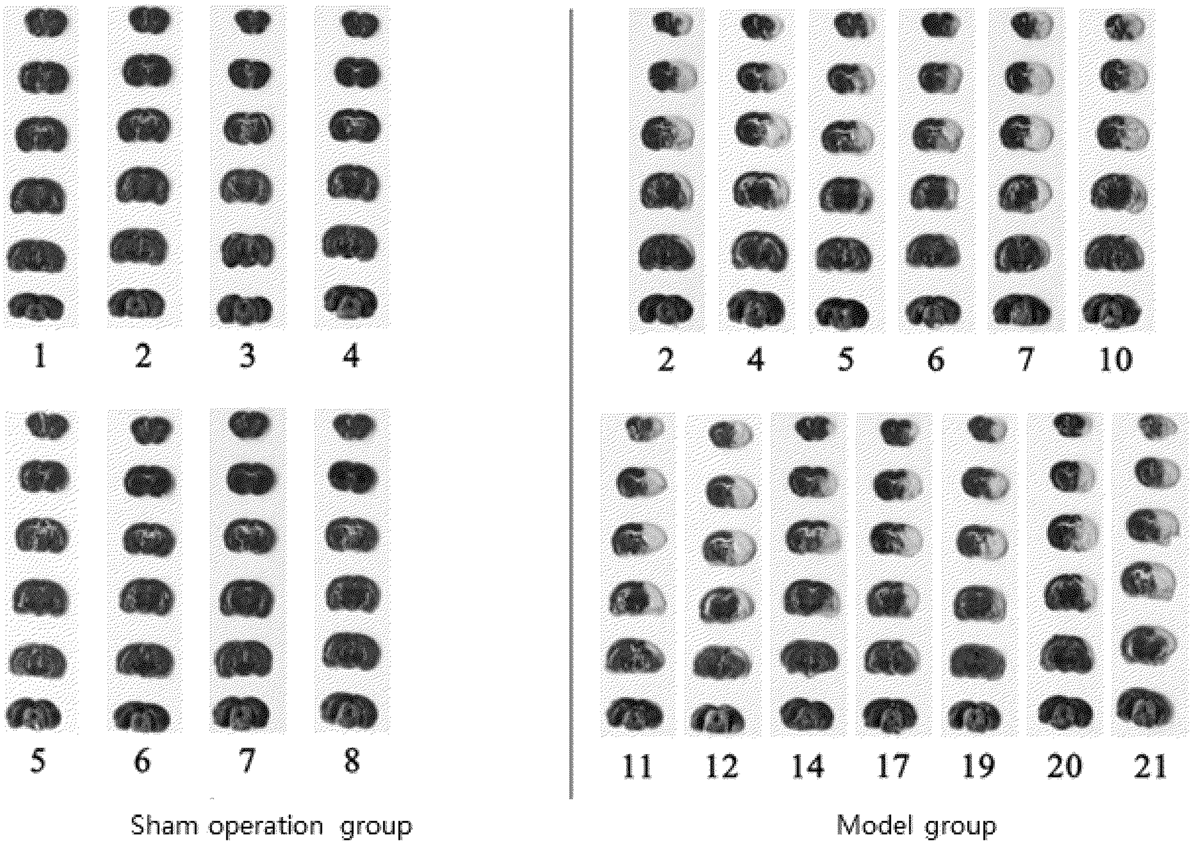
1
2
3
4
5
6
7
8
Sham operation group
2
4
5
6
7
10
11
12
14
17
19
20
21
Model group

Figure 21

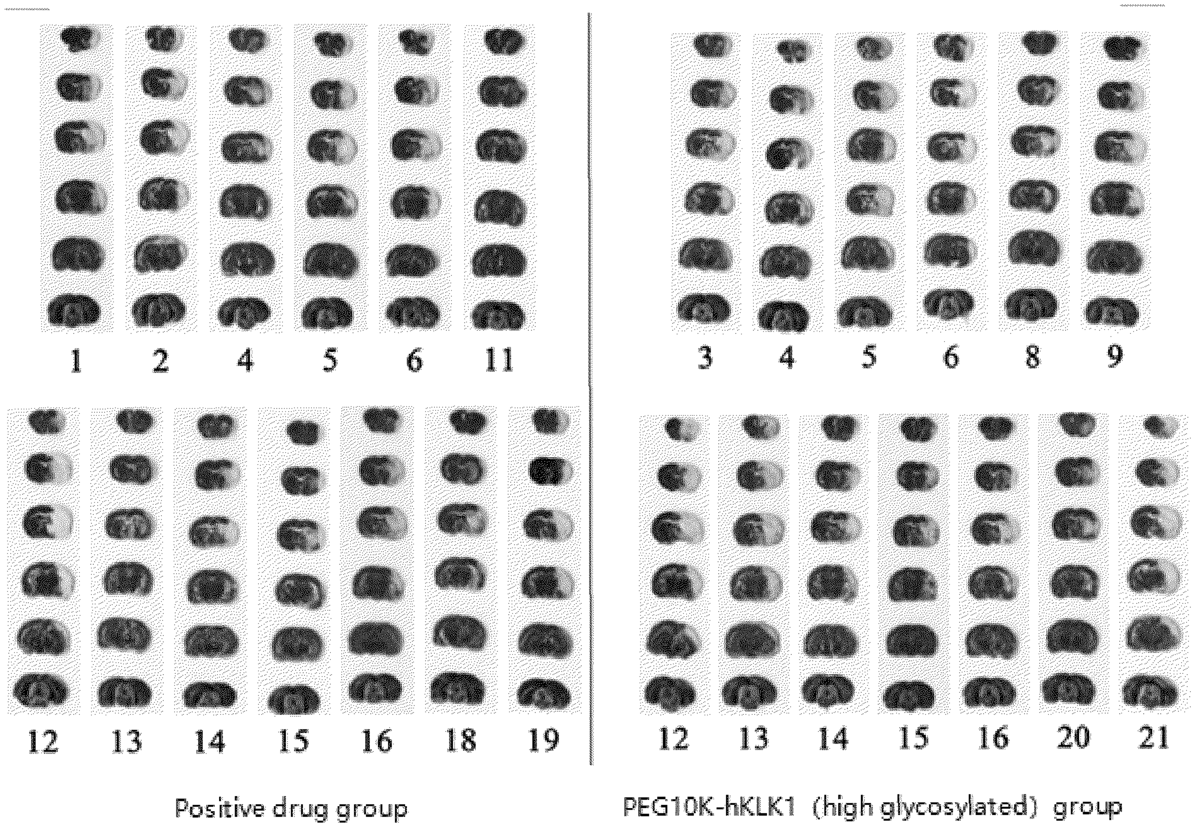


Figure 22

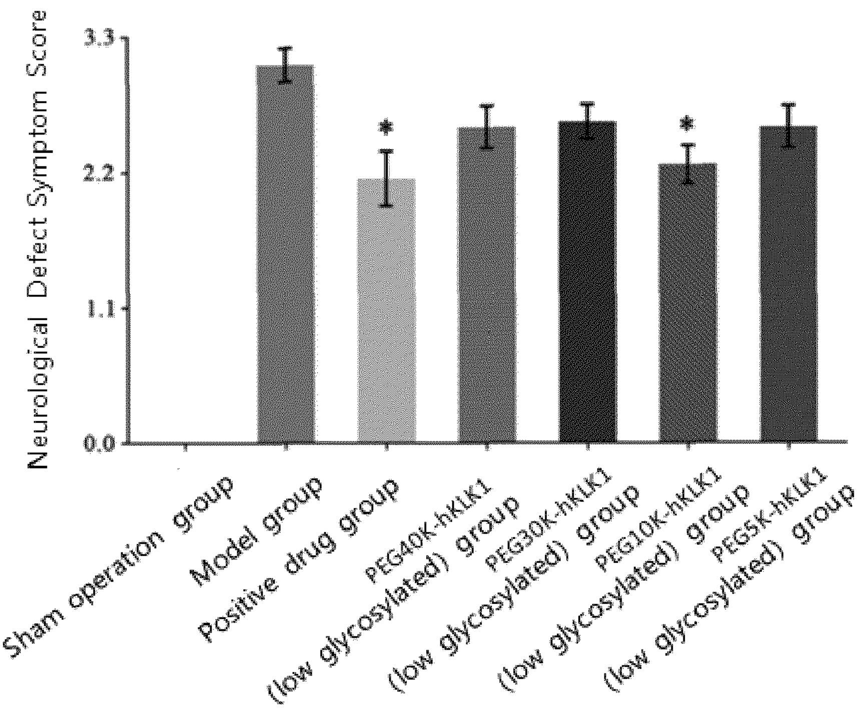


Figure 23

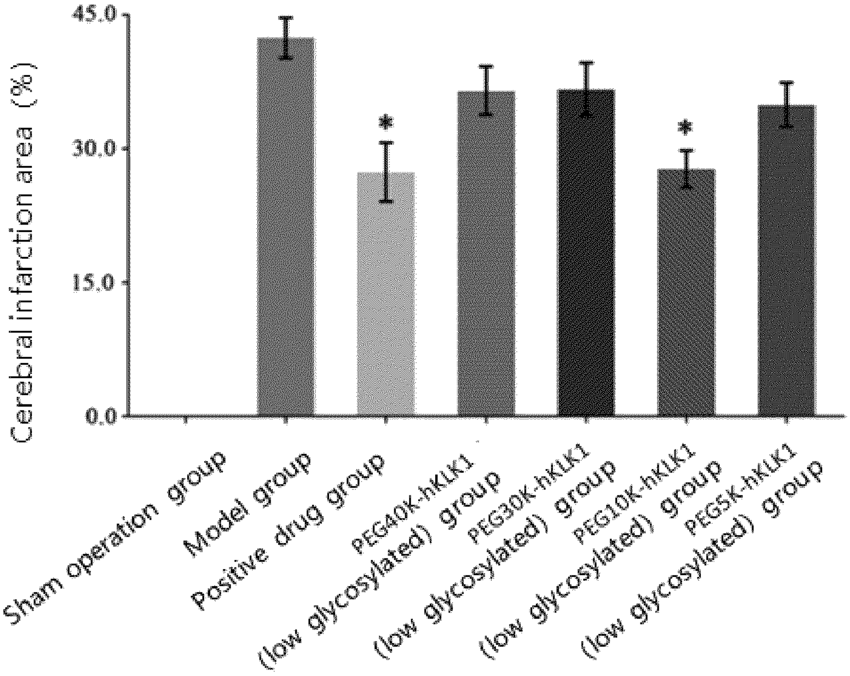


Figure 24

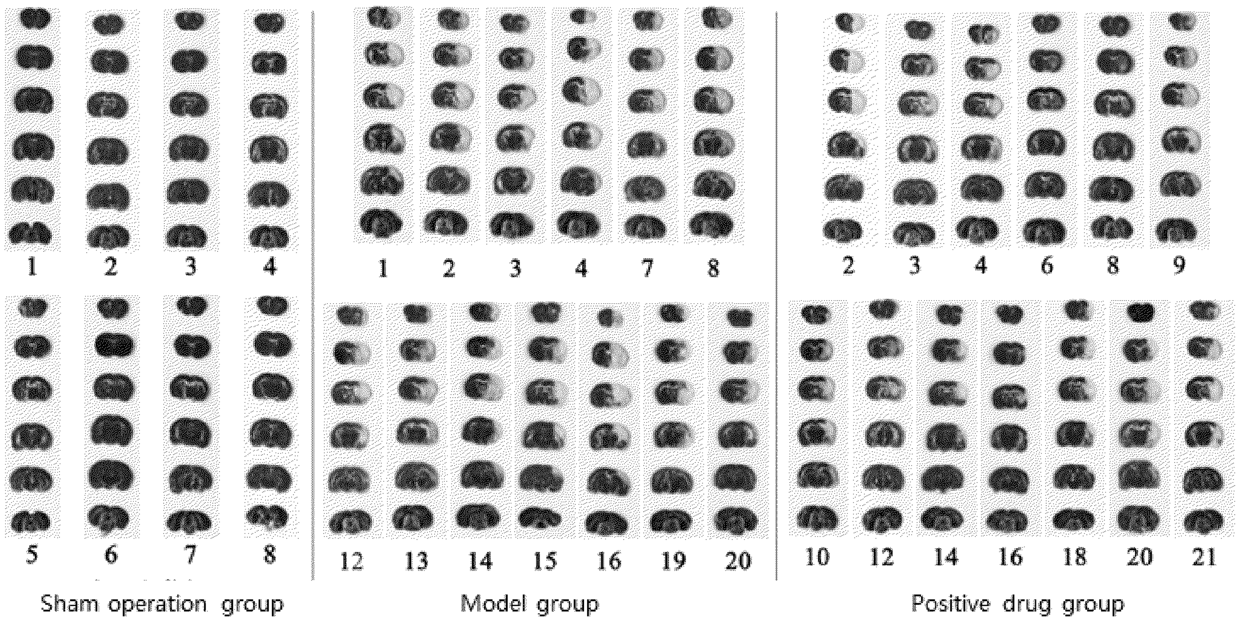


Figure 25

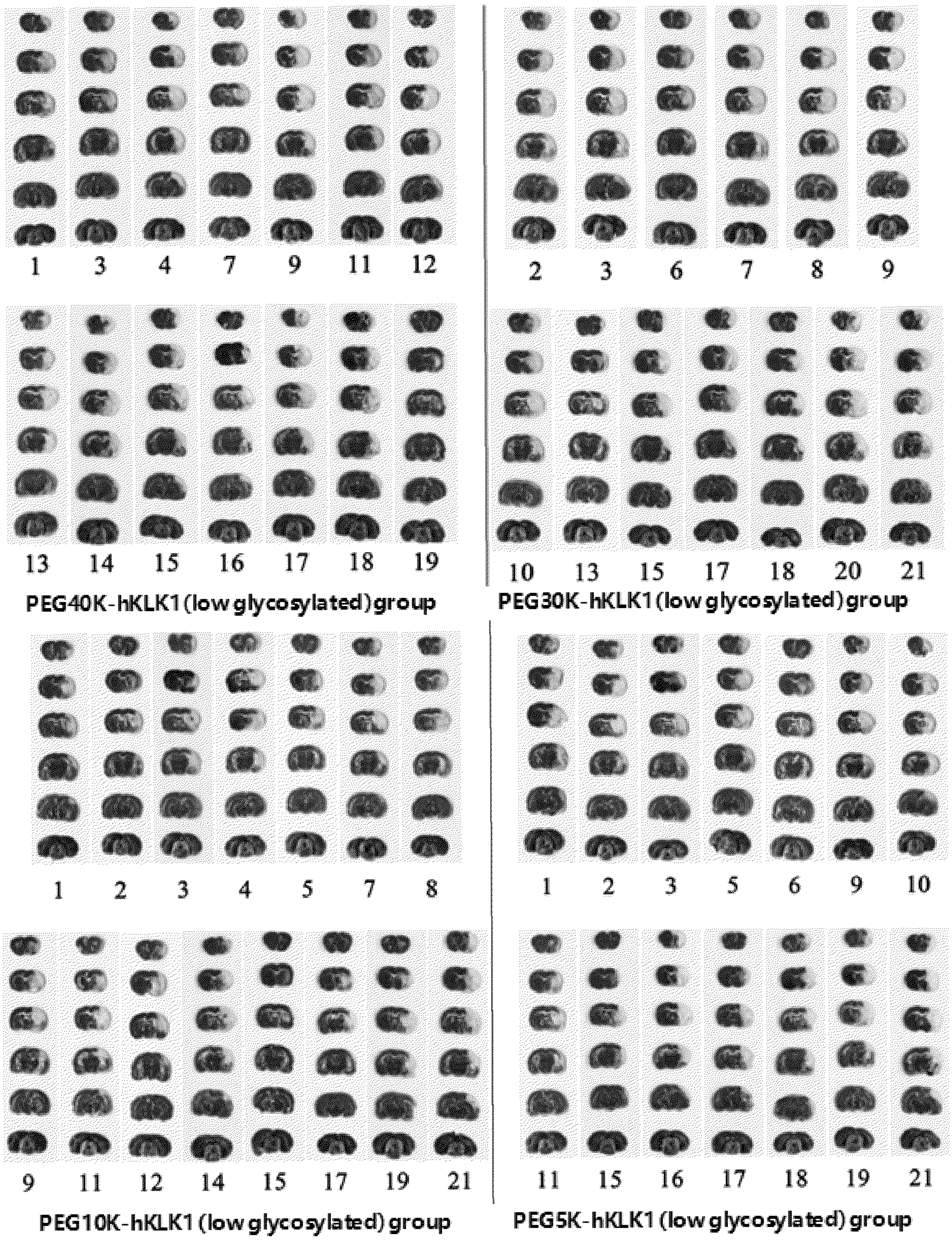
1 3 4 7 9 11 12
13 14 15 16 17 18 19
PEG40K-hKLK1 (low glycosylated) group
2 3 6 7 8 9
10 13 15 17 18 20 21
PEG30K-hKLK1 (low glycosylated) group
1 2 3 4 5 7 8
9 11 12 14 15 17 19 21
PEG10K-hKLK1 (low glycosylated) group
1 2 3 5 6 9 10
11 15 16 17 18 19 21
PEG5K-hKLK1 (low glycosylated) group

Figure 26

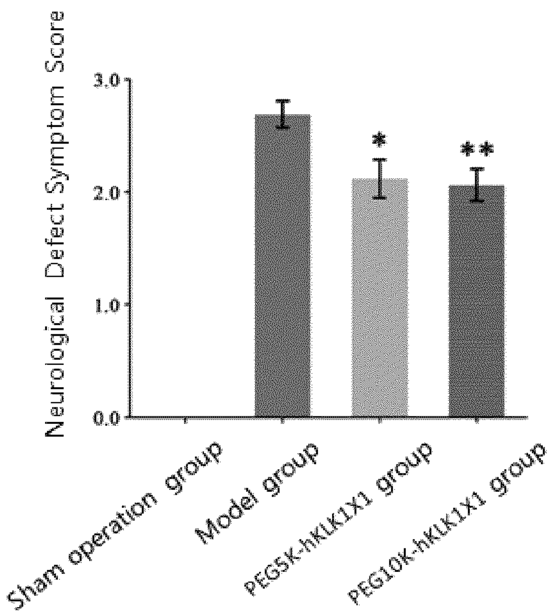


Figure 27

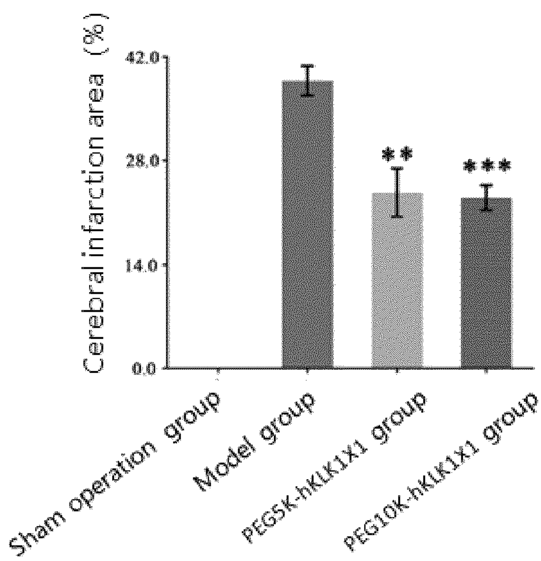


Figure 28

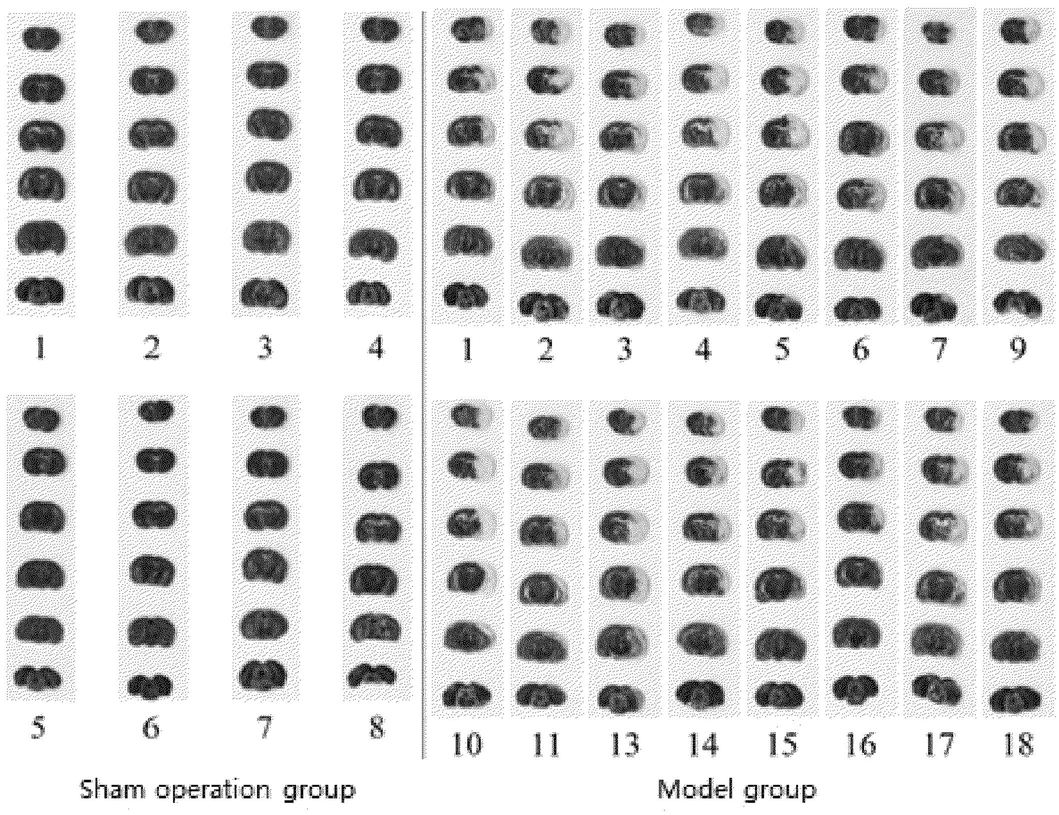


Figure 29

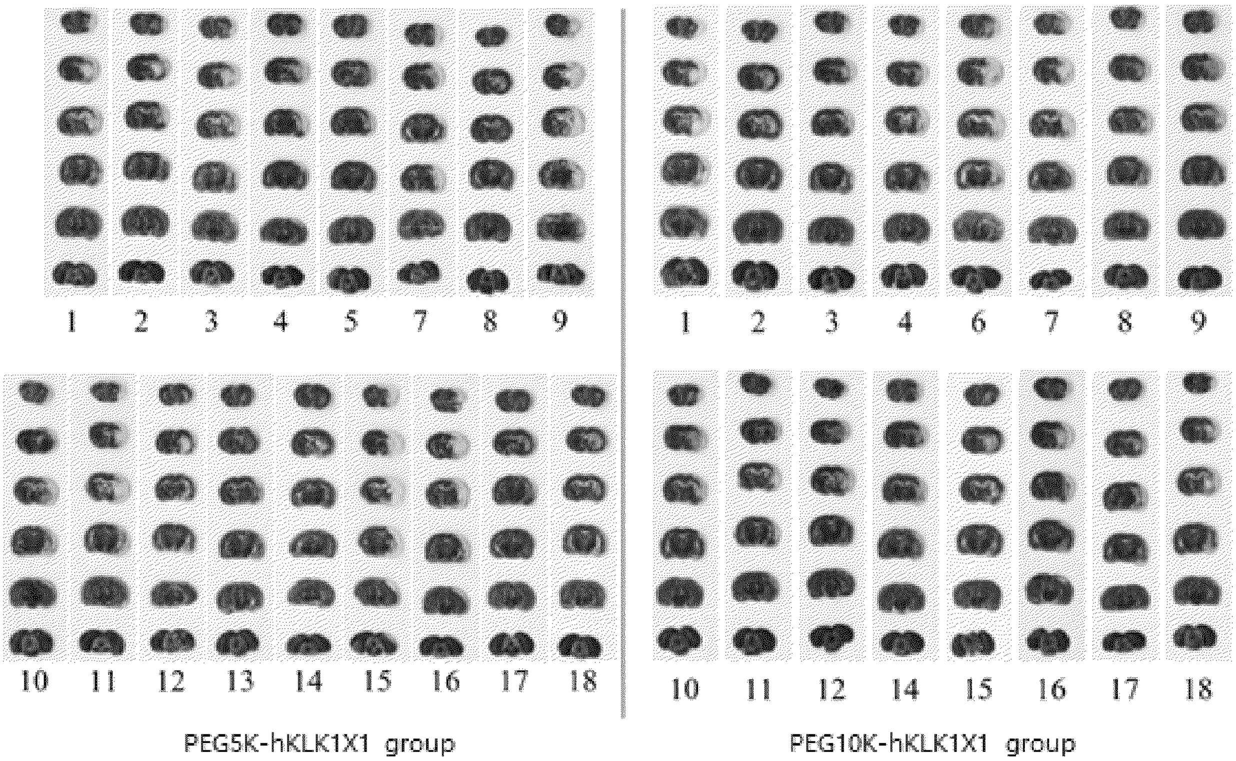


Figure 30

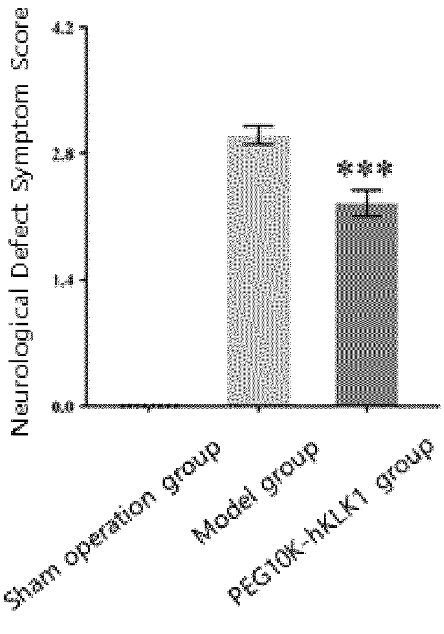


Figure 31

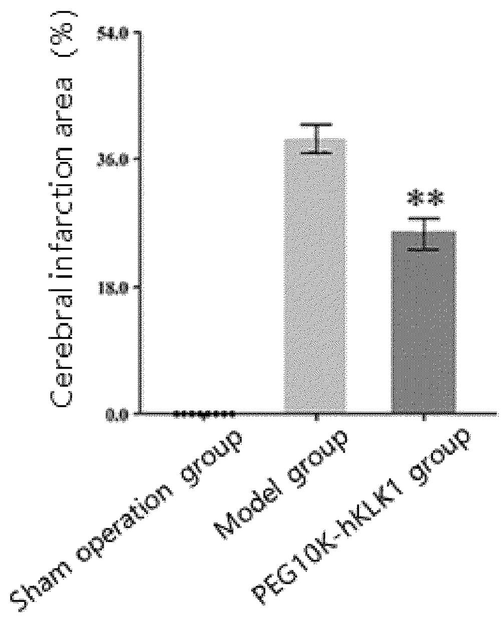


Figure 32

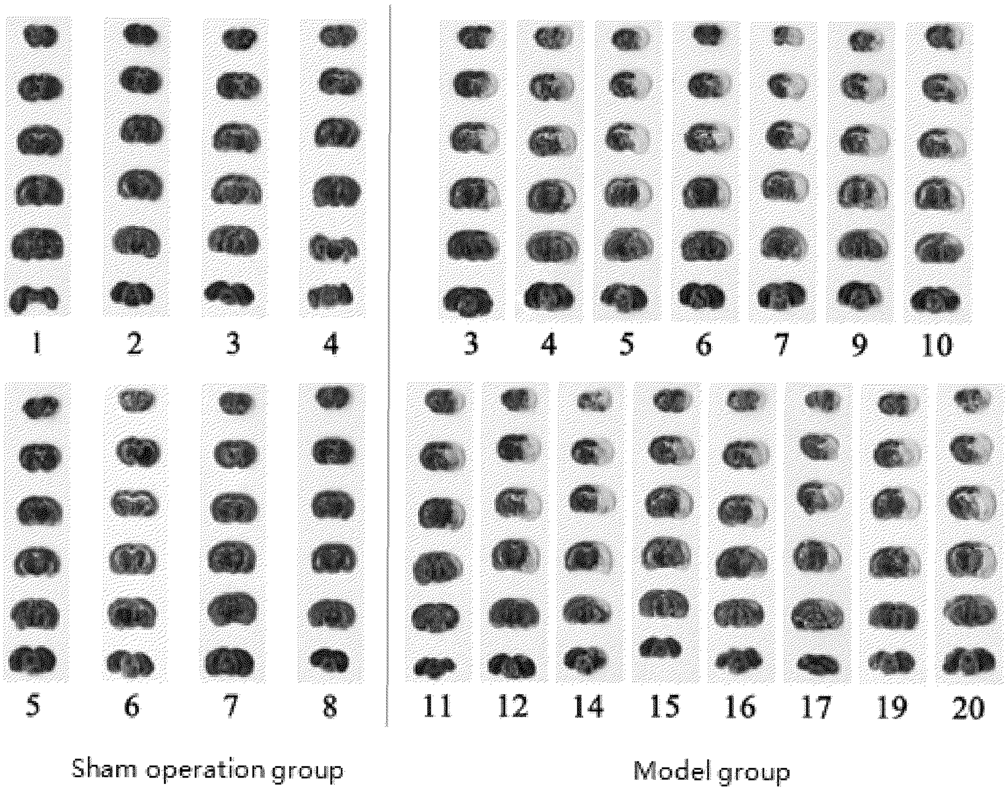


Figure 33

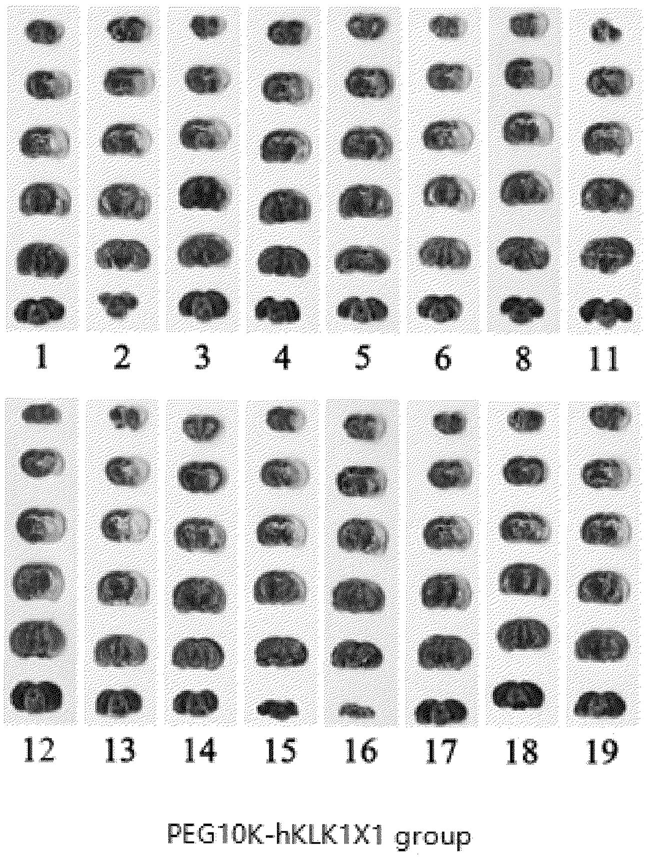


Figure 34

## INTERNATIONAL SEARCH REPORT

International application No.
**PCT/CN2022/132102**

**A. CLASSIFICATION OF SUBJECT MATTER**

C12N 9/64(2006.01)i;C12N 9/96(2006.01)i;A61K 38/48(2006.01)i;A61P 9/10(2006.01)i;A61P 7/02(2006.01)i;A61P 25/02(2006.01)i;A61P 13/12(2006.01)i

According to International Patent Classification (IPC) or to both national classification and IPC

**B. FIELDS SEARCHED**

Minimum documentation searched (classification system followed by classification symbols)

C12N,A61K,A61P

Documentation searched other than minimum documentation to the extent that such documents are included in the fields searched

Electronic data base consulted during the international search (name of data base and, where practicable, search terms used)

CNABS, DWPI, SIPOABS, CNTXT, WOTXT, EPTXT, USTXT, CNKI, ISI WEB OF SCIENCE, PUBMED, 万方数据库, WANFANG DATA: 激肽释放酶I, 低糖基化, 聚乙二醇, 突变体, KLK1, underglycosylation, mutant, NMS, NHT, NFS; EBI +GENBANK+中国专利生物序列检索平台, Chinese Patent Biological Sequence Retrieval Platform: 对SEQ ID NOs.3-6进行的序列检索, Search for Sequence SEQ ID NOs.3-6. .

**C. DOCUMENTS CONSIDERED TO BE RELEVANT**

| Category* | Citation of document, with indication, where appropriate, of the relevant passages | Relevant to claim No. |
|---|---|---|
| X | US 2013323222 A1 (MATTHEW, C. et al.) 05 December 2013 (2013-12-05)<br>see claims 1-5, and description, paragraphs 42, 47, and 113 | 1-12 |
| Y | US 2013323222 A1 (MATTHEW, C. et al.) 05 December 2013 (2013-12-05)<br>see claims 1-5, and description, paragraphs 42, 47, and 113 | 13-17 |
| Y | CN 107760661 A (ZONHON BIOPHARMA INSTITUTE, INC.) 06 March 2018 (2018-03-06)<br>see claims 1, 4, 5, and 8 | 13-17 |
| Y | CN 104073482 A (ZONHON BIOPHARMA INSTITUTE, INC. et al.) 01 October 2014 (2014-10-01)<br>see claims 1-10 | 13-17 |
| A | CN 107058269 A (ZONHON BIOPHARMA INSTITUTE, INC.) 18 August 2017 (2017-08-18)<br>see abstract | 1-17 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| | **14 February 2023** |
| Name and mailing address of the ISA/CN<br>**China National Intellectual Property Administration (ISA/CN)**<br>**China No. 6, Xitucheng Road, Jimenqiao, Haidian District, Beijing 100088**<br>Facsimile No. **(86-10)62019451** | Authorized officer<br><br>Telephone No. |

Form PCT/ISA/210 (second sheet) (July 2022)

**INTERNATIONAL SEARCH REPORT**

International application No.
**PCT/CN2022/132102**

**C. DOCUMENTS CONSIDERED TO BE RELEVANT**

| Category* | Citation of document, with indication, where appropriate, of the relevant passages | Relevant to claim No. |
|---|---|---|
| A | WO 2009039704 A1 (SHANGHAI WANXING BIOPHARMACEUTICALS, CO., LTD. et al.) 02 April 2009 (2009-04-02)<br>see abstract, and claims 1 and 10 | 1-17 |
| A | CN 101134952 A (GUANGDONG TIANPU BIOLOGY CHEMICAL MEDICAL CO., LTD.) 05 March 2008 (2008-03-05)<br>see abstract | 1-17 |
| A | 黄秀东等 (HUANG, Xiudong et al.). "人激肽释放酶-1在甲醇酵母中高水平表达、纯化与鉴定 (High Level Expression, Purification and Characterization of Human Kallikrein-1 in Pichia pastoris)"<br>*生物工程学报 (Chinese Journal of Biotechnology),*<br>Vol. 24, No. 7, 25 July 2008 (2008-07-25),<br>ISSN: 1000-3061,<br>pages 1186-1193, see abstract | 1-17 |
| A | TANG J et al. "Expression, Crystallization, and Three-dimensional Structure of the Catalytic Domain of Human Plasma Kallikrein"<br>*The Journal of Biological Chemistry,* Vol. vol. 280, No. 49, 09 December 2005 (2005-12-09),<br>ISSN: 1083-351X,<br>pages 41077-41089, see abstract | 1-17 |

Form PCT/ISA/210 (second sheet) (July 2022)

**INTERNATIONAL SEARCH REPORT**

International application No.
**PCT/CN2022/132102**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2022/132102**

| Patent document cited in search report | Publication date (day/month/year) | Patent family member(s) | Publication date (day/month/year) |
|---|---|---|---|
| US 2013323222 A1 | 05 December 2013 | EP 2854841 A1 | 08 April 2015 |
| | | EP 2854841 A4 | 09 March 2016 |
| | | EP 2854841 B1 | 22 February 2017 |
| | | ES 2625548 T3 | 19 July 2017 |
| | | HUE 032613 T2 | 30 October 2017 |
| | | WO 2013181755 A1 | 12 December 2013 |
| | | US 2017119862 A1 | 04 May 2017 |
| | | US 9839678 B2 | 12 December 2017 |
| | | LT 2854841 T | 12 June 2017 |
| | | CY 1119073 T1 | 10 January 2018 |
| | | SI 2854841 T1 | 30 June 2017 |
| | | CA 2880085 A1 | 12 December 2013 |
| | | CA 2880085 C | 07 September 2021 |
| | | HRP 20170673 T1 | 14 July 2017 |
| | | US 2015196624 A1 | 16 July 2015 |
| | | US 9364521 B2 | 14 June 2016 |
| | | PT 2854841 T | 15 May 2017 |
| | | PL 2854841 T3 | 31 August 2017 |
| | | DK 2854841 T3 | 22 May 2017 |
| CN 107760661 A | 06 March 2018 | None | |
| CN 104073482 A | 01 October 2014 | WO 2015100768 A1 | 09 July 2015 |
| | | US 2017049864 A1 | 23 February 2017 |
| | | US 10052368 B2 | 21 August 2018 |
| CN 107058269 A | 18 August 2017 | None | |
| WO 2009039704 A1 | 02 April 2009 | None | |
| CN 101134952 A | 05 March 2008 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 107058269 A
- CN 101134952 A
- CN 101134953 A
- US 20130323222 A
- CN 101092598 A